# EUROPEAN PATENT APPLICATION

(11) **EP 2 353 636 A1**
(43) Date of publication of application: **10.08.2011**
(21) Application number: 10152798.4
(22) Date of filing: 05.02.2010
(51) Int. Cl.: A61N 1/05, A61B 5/04, A61B 5/0478, H01L 21/70, A61N 1/36

(54) **Neurological interfacing probe**

(71) Applicant: Max-Planck-Gesellschaft zur Förderung der Wissenschaften e.V., 80539 München (DE)
(72) Inventor: The designation of the inventor has not yet been filed
(74) Representative: Jordan, Volker Otto Wilhelm

(57) **Abstract**

For a neurological interfacing probe (10; 10'), having
- a contacting section having a plurality of contacts for electrically connecting the neurological interfacing probe (10; 10') and
- an elongated impalement section (12; 12') extending from the contacting section and being adapted for impaling into neuronal tissue (18) to be interfaced;
- wherein the impalement section (12; 12') has a plurality of interaction sites (14; 14') which each are electrically connected with at least one respective contact of said plurality of contacts by means of at least one respective interconnecting trace (16; 16') extending along the impalement section (12; 12') and which are adapted to electrically interact with the neuronal tissue (18);

it is proposed that the plurality of interaction sites (14; 14') are provided by semiconductor structures which are formed in semiconductor material and which are adapted to provide at least one of transistor functionality and capacitor functionality for electrically interacting with the neuronal tissue (18). Additionally and alternatively it is proposed that the interaction sites (14; 14') are covered by at least one insulating layer electrically isolating the interaction sites (14; 14') from the neuronal tissue (18) to provide capacitive coupling between the respective interaction sites (14; 14') and the neuronal tissue (18).

## Description

The electrical interfacing of brain tissue is a topical issue in brain physiology, neuroprosthetics and brain-computer interfacing (Donoghue, 2002; Schwartz et al., 2006; Lebedev & Nicolelis, 2006). Generally, metal electrodes are used such as thin wires (Kralik et al., 2001; Nicolelis et al., 2003) or planar contacts on a silicon substrate (Wise et al., 1970; Normann et al., 1999; Ensell et al., 2000; Miller et al., 2004; Hochberg et al., 2006). Recordings of local field potentials as well as of single unit activity are applied to control prosthetic devices (Donoghue, 2002; Schwartz et al., 2006). An optimization of the tissue/electrode interaction is important to achieve strong and reliable contacts. In experiments with cultured neurons as with brain slices, it has been shown that a direct interfacing to semiconductor devices is most effective with transistor recording and with capacitor stimulation (Voelker & Fromherz, 2005; Hutzler & Fromherz; 2004; Schoen & Fromherz, 2008; Stangl & Fromherz, 2008)

The present invention, according to a first aspect, relates to a neurological interface probe, having a contacting section having a plurality of contacts for electrically connecting the neurological interfacing probe and an elongated impalement section extending from the contacting section and being adapted for impaling into neuronal tissue to be interfaced, wherein the impalement section has a plurality of interaction sites which each are electrically connected with at least one respective contact of said plurality of contacts by means of at least one respective interconnecting trace extending along the impalement section and which are adapted to electrically interact with the neuronal tissue, and wherein at least the impalement section comprises semiconductor material.

Further, the present invention, according to a second aspect, relates to a neurological interface probe having a contacting section having a plurality of contacts for electrically connecting the neurological interfacing probe and an elongated impalement section extending from the contacting section and being adapted for impaling into neuronal tissue to be interfaced, wherein the impalement section has a plurality of interaction sites which each are electrically connected with at least one respective contact of said plurality of contacts by means of at least one respective interconnecting trace extending along the impalement section and which are adapted to electrically interact with the neuronal tissue.

The functioning of the human brain is based on the interaction of a large number of neurons. In order to understand such neurological processes, it is necessary to measure the neurological activity of the neuronal tissue, e.g. brain tissue at an high spatial resolution. These requirements can only be fulfilled by neurological probes which can be implanted into the neuronal tissue to be analysed. Beginning in the 1950s, the use of microelectrodes together with electronic recording and signal processing began to allow meaningful studies of the central nervous system at a cellular level. In this way, significant progress could be achieved in understanding the functioning of single neurons. Developments in other technical fields, for example the development of integrated sensors using microelectromechanical system (MEMS) techniques, allowed further improvements.

Such logical probes are needed with respect to all electrical activities, in particular including action potentials of single cells (single unit activity), action potentials of small cell groups (multi unit activity) and the supervision of many action potentials also of neighbouring regions (local field potential). In the following, it is not distinguished between single unit activity, multi unit activity and local field potential, but always referred to action potential or action potentials.

The most advanced probes are based on above-mentioned MEMS techniques which allow manufacturing of 3D-probe structures out of silicon by combining method techniques for manufacturing micro chips and controlled deposition of material (micro machining) in order to built sensors, electric circuits, mechanical elements and controllers. Moreover, based on these techniques, neurological probes having a tip shape can be manufactured which can easily be impaled into the (brain) tissue to be analysed.

One prominent representative of such a neurological probe is the so called "Michigan probe" which is based on a long conical silicon carrier on which metal electrodes are formed by means of lithographic processes. Such probes can easily be manufactured, since simple lithographic processes can be used for forming the metal electrodes.

In particular, a major disadvantage of such probes utilizing metal electrodes for interaction with the neuronal tissue lies in the fact that the electrical coupling between the metal electrodes and the neuronal tissue is reduced over a longer period such that electrical signals detected by the electrodes decrease. This effect is caused by a defence reaction of the neuronal tissue when the neurological probe is introduced into the tissue, as the probe material acts as a contaminant to the tissue. As a consequence of such a defence reaction, glia cells are produced such that the impaled probe is encapsulated and therefore electrically isolated from the tissue.

Moreover, as no effective electrical isolation between the metal electrodes and neuronal tissue or the electrolyte solution in which the tissue is embedded or which is present in the neuronal tissue exists, a charge exchange on the boundary surface between metal electrodes and the neuronal tissue or electrolyte solution is caused. By means of such a charge exchange (which is usually called "Faraday current"), severe damaging of the neuronal tissue can occur, for example by changes of the pH-value of the tissue caused by the faraday current between the electrode and the electrolyte solution.

As a consequence, silicon based implantable semiconductor probes utilizing metal electrodes for interaction with the neuronal tissue to be probed are not suited for an *in vivo* long term analysis of neuronal tissue.

Further, such probes utilizing metal electrodes fabricated using simple lithographic processes have limited potential for integrating further functionality such as integrated signal preprocessing, into the neurological interfacing probe.

In view of these considerations, it is an object of the present invention to provide a neurological interfacing probe allowing an improved coupling between the probe and the neuronal tissue (possibly brain tissue of a living brain) to be interfaced.

Further, it is an object of the present invention to provide a neurological interfacing probe having an improved degree of bio-compatibility, preferably long-term bio-compatibility, with the neuronal tissue (possibly brain tissue of a living brain) to be interfaced.

Further, it is an object of the present invention to provide a neurological interfacing probe allowing for a coupling between the probe and the neuronal tissue to be interfaced without direct charge exchange, i.e. without occurrence of so-called "Faraday currents".

Further, it is an object of the invention to provide a neurological interfacing probe which with regard to its structure and available manufacturing techniques has the potential to integrate additional signal processing functionalities into the probe, in particular in at least one of its contacting section and its impalement section.

At least one of those objects is achieved by a neurological interfacing probe according to the first aspect of the invention, which comprises:
- a contacting section having a plurality of contacts for electrically connecting the neurological interfacing probe and
- an elongated impalement section extending from the contacting section and being adapted for impaling into neuronal tissue to be interfaced;
- wherein the impalement section has a plurality of interaction sites which each are electrically connected with at least one respective contact of said plurality of contacts by means of at least one respective interconnecting trace extending along the impalement section and which are adapted to electrically interact with the neuronal tissue, and
- wherein at least the impalement section comprises semiconductor material.

According to the invention in its first aspect, the plurality of interaction sites are provided by semiconductor structures which are formed in the semiconductor material and which are adapted to provide at least one of transistor functionality and capacitor functionality for electrically interacting with the neuronal tissue.

By means of such transistor functionality, also weak electrical signals, electrical fields and in particular so-called action potentials within the neuronal tissue can be detected and analysed, on the basis of the strength of an electrical current provided in the semiconductor structures, which is influenced by said electrical fields or potentials. In this respect, an amplification effect provided by such transistor functionalities can be used for measuring even very weak signals.

Moreover, by the provision of semiconductor structures formed in the semiconductor material which are adapted to provide capacitor functionality for electrically interacting with the neuronal tissue, a direct charge exchange between the interaction sites of the neurological interfacing probe and the neuronal tissue can be avoided. In this way, bio-compatibility of the neurological interfacing probe with the tissue to be analysed can be significantly improved. Therefore, even long-term measurements are possible without damaging the brain tissue during the measurement period.

Preferably, the interaction sites are covered by at least one insulating layer electrically isolating the interaction site from the neuronal tissue. By the provision of at least one insulating layer electrically isolating the interaction site from the neuronal tissue, an exclusively capacitive coupling between the interaction sites and the neuronal tissue is ensured. Therefore, as already mentioned, a direct charge exchange between the interaction sites of the neurological interfacing probe and the neuronal tissue is avoided by means of such an insulating layer. Consequently, the bio-compatibility of the neurological interfacing probe with the tissue to be analysed can be significantly improved such that even long term measurements are possible without damaging the brain tissue during the measurement period. Preferably, a highly bio-compatible material is used for providing the insulating layer or at least a surface layer which comes into contact with the neuronal tissue.

Preferably, the whole impalement section is covered with such at least one insulating layer.

Further, at least one of above-given objects is achieved by a neurological interfacing probe according to the second aspect of the invention, which comprises:
- a contacting section having a plurality of contacts for electrically connecting the neurological interfacing probe and
- an elongated impalement section extending from the contacting section and being adapted for impaling into neuronal tissue to be interfaced;
- wherein the impalement section has a plurality of interaction sites which each are electrically connected with at least one respective contact of said plurality of contacts by means of at least one respective interconnecting trace extending along the impalement section and which are adapted to electrically interact with the neuronal tissue.

According to the invention in its second aspect, the interaction sites are covered by at least one insulating layer electrically isolating the interaction sites from the neuronal tissue to provide capacitive coupling between the respective interaction site and the neuronal tissue.

As discussed above, a direct charge exchange between the interaction sites of the neurological interfacing probe and the neuronal tissue can be avoided by means of such an insulating layer leading to substantial advantages. Preferably, the whole impalement section is covered with such at least one insulating layer.

Preferably, at least one of the intersection sites is adapted to provide transistor functionality for electrically interacting with the neuronal tissue by means of said capacitive coupling. Further, it is proposed that at least one of said interaction sites is adapted to provide capacitor functionality for electrically interacting with the neuronal tissue by means of said capacitive coupling. In this respect, it is further proposed that at least one interaction site is provided by a semiconductor structure which is formed in the semiconductor material and which is adapted to provide at least one of transistor functionality and capacitor functionality for electrically interacting with the neuronal tissue. By means of those proposals for the neurological interfacing probe according to the second aspect of the invention, the advantages discussed in the foregoing with respect to the first aspect of the invention can be achieved.

In order to facilitate the implantation of the neurological interfacing probe into neuronal tissue, in a preferred embodiment the impalement section comprises an impalement needle or impalement pin or impalement shank which has a free tip end. In this way, damaging of the tissue by impaling the neurological interfacing probe into the tissue can be minimized.

In order to simplify the manufacturing process of the neurological interfacing probe, it is proposed to form the interaction sites by semiconductor device manufacturing techniques comprising at least one of masking, doping, implanting, lithographic patterning, etching, covering with the respective layer of other material, at least locally removing material and surface oxidizing. In this way, a variety of semiconductor techniques can be used for manufacturing of the interaction sites.

Preferably, the impalement section is formed by etching semiconductor material. In this way, formation of the desired shape of the impalement section at an high accuracy can be ensured.

Preferably, the impalement section and the contacting section are integrally formed by etching semiconductor material. In this way, the manufacturing process of the neurological interfacing probe can be facilitated, thereby reducing manufacturing effort and costs.

Preferably, at least a subset of said interaction sites is provided in the form of field effect transistors having at least a drain section, a channel section, and a source section, wherein the drain section and the source section are connected with a respective contact of the contacting section by means of a respective interconnecting trace, and wherein the channel section or a gate section of the field effect transistor serves to electrically couple the field effect system with a neuronal tissue. In this way, even small electric fields going along with action potentials excited in the neuronal tissue can be detected, as these electric fields act like the electric fields associated with a gate voltage applied to the channel section of the field effect transistor, typically (but not necessarily) via a gate section of the field effect transistor, so that the source-drain current is influenced and modulated by field changes. In this way, a neurological interfacing probe having a good sensitivity is obtained.

Further, it is proposed that at least a subset of said interaction sites is provided in the form of electrode sections which are connected with a respective contact of the contacting sections by means of a respective interconnecting trace, and which are adapted to serve as capacitor for electrical coupling with the neuronal tissue. In this way, by charging and discharging the electrode sections, a capacitive change of the electrical potential in the neuronal tissue can be induced without accompanying faraday currents.

It is further proposed that the electrodes sections or/and the drain sections or/and the source sections are provided with highly doped silicon conductor material sections arranged between less highly doped or differently doped semiconductor material. Further, also the interconnecting traces can be provided by highly doped semiconductor material sections arranged between less highly doped or differently doped semiconductor material. Accordingly, additional structuring steps for providing metal contacts and metal interconnects and the like can be avoided. Further, it is believed that it is advantageous for certain measuring situations if metal subsections of the impalement section are avoided. This can be an advantage e.g. for nuclear magnetic resonance (NMR) measurements effected simultaneously.

In a third aspect, the present invention relates to a method for manufacturing the neurological interfacing probe according to the first or/and the second aspect of the invention. According to the third aspect of the invention, the contacting section and the impalement section are integrally etched from a semiconductor substrate after the formation of the semiconductor structures. In this way, manufacturing of the neurological interfacing probe is significantly facilitated.

Preferably, the semiconductor substrate has an etch stop layer for at least one etching step, preferably at least one of a first and second etching step. In this respect it is further proposed that the semiconductor substrate is a Silicon-on-Insulator (SOI) wafer having a buried oxide layer which serves as etch stop layer. This allows for the application of a multi-step etching process carried out on both sides of the semiconductor substrate. In this way, the etching process can be optimized.

In a preferred embodiment, the contacting section and the impalement section are etched using a multi-step-etching process, with at least a first etching step being performed on a first side of the semiconductor substrate and at least a second etching step being performed on a second side of the semiconductor substrate, on which the semiconductor structures are formed. Independently performing at least a first etching step and at least a second etching step on different sides of the semiconductor substrates allows to use optimized etching methods and gives many options for the structure of the neurological interfacing probe to be obtained.

Preferably, the first etching step is based on an wet etching process. In a particularly preferred embodiment, the first etching step is based on an EDP etching process. In this way, an anisotropic etching (in which the etching process can be stopped by e.g. oxide intermediate layers) becomes possible, which can be advantageous for example if the thickness of the semiconductor substrate is large.

Further, the second etching step can based on a dry etching process. Preferably, the second etching step is based on a plasma etching process. In this way, etching at an improved accuracy can be ensured. Moreover, an undesired undercut can be avoided.

To advantage, a multi-step structuring process can be carried out on a/the second side of the semiconductor substrate, preferably preceding a/the first etching step, wherein the multi-step structuring process comprises at least one of the following substeps:
S1) implantation of a dopant on the second side of the
   semiconductor substrate such that a plurality of highly doped semiconductor sections are formed within the semiconductor substrate;
S2) formation of a silicon dioxide layer on the second side of the semiconductor substrate followed by partial etching of the silicon dioxide layer such that a silicon oxide section serving as gate oxide of a field effect transistor is formed from the silicon dioxide layer;
S3) deposition of an insulating layer on the second side of the semiconductor substrate;
S4) deposition of a metal layer on the second side of the semiconductor substrate followed by partial removal of the metal layer such that metal sections are formed from the metal layer serving as contacts.

It is further suggested that a/the first etching step is part of a multi-step processing of the semiconductor substrate which comprises at least one of the following steps:
F1) deposition of a protection layer, preferably a polyimide layer, on the second side of the semiconductor substrate;
F2) deposition of a silicon dioxide layer on the first side of the semiconductor substrate followed by partial etching of the silicon dioxide layer such that a silicon dioxide section partially covering the semiconductor substrate is formed;
F3) partial removal of the semiconductor substrate by etching, preferably wet etching, such that the buried etch stop layer is exposed on the first side after removal of the semiconductor substrate;
F4) removal of the protection layer if deposited in step F1).

To advantage, the second etching step can be part of a multi-step processing of the semiconductor substrate which comprises at least one of the following steps:
G1) deposition of a protection layer, preferably a polyimide layer, on
   top of the second side of the semiconductor substrate;
G2) removal of the semiconductor substrate by plasma etching such that the buried etch stop layer is exposed on the second side after removal of the substrate;
G3) removal of the protection layer if deposited in step G1);
G4) separation of the neurological interfacing probe by removing or braking the etch stop layer.

In a fourth aspect, the present invention relates to a method for measuring brain activity or/and for inducing brain activity in a brain, preferably in the brain of a living creature. According to the intervention, the method comprises the step of impaling the impalement section of the neurological interfacing probe according to the first or/and second aspect of the invention in the brain and the steps of measuring or/and inducing action potentials of neuronal tissue of the brain by electrical means using the contacts of the contacting section.

In the following, the invention in its different aspects is further explained and exemplified on basis of illustrative embodiments and with reference to the attached figures.
- Fig. 1: shows a top view of a neurological interfacing probe of the present invention according to a first embodiment which is adapted to act as a stimulating probe.
- Fig. 2: shows a top view of a neurological interfacing probe of the present invention according to a second embodiment which is adapted to act as a recording probe.
- Fig. 3: shows the neurological interfacing probes according to the first and second embodiment when impaled into neuronal tissue.
- Figs. 4a to 4k: show subsequent processing steps of a method of the present invention for manufacturing the neurological interfacing probe of the present invention.
- Fig.5: shows a measurement setup for measuring the coupling

- Fig.6: between slices of a brain and the neurological interfacing probe according to the present invention. partially shows the setup of Figure 5 modified such that the coupling with single neurons 120', 121' instead of brain slices is measured.
- Fig.7: shows diagrams (a) to (c) demonstrating the successful recording of action potentials excited in the setup of figure 6.
- Fig.8: shows a diagram demonstrating the successful stimulation of action potentials in the setup of figure 6.
- Fig.9: shows a diagram demonstrating the successful recording of a field potential in a brain slice measured in the setup of figure 5.
- Fig.10: shows a schematic cross section of acute brain slice with impaled transistor needle chip (TNC) serving as neurological interfacing probe. A nylon net holds the slice on an Agar layer in artificial cerebrospinal fluid (ACSF). The surface layers of the slice are damaged by the cutting process (dashed). The bath is held at ground potential with an Ag/AgCl electrode. The slice is stimulated with an impaled tungsten electrode (TE).
- Fig.11: shows a micrograph of such a transistor needle chip (TNC). The micrograph shows the kinked structure of a TNC with a thin needle and the transistors and with a contact plate where the drain contact (aluminum) of the first transistor is visible. The insert shows a scanning electron micrograph of the needle tip with the circular areas (diameter 20 µm) corresponding to the locations of four transistor interaction sites.
- Fig.12: shows acute brain slice from rat hippocampus. Fig. 12A shows a slice that is held on an agar layer with a nylon net with impaled transistor needle chip (TNC) and impaled tungsten electrode (TE). Fig. 12B is a picture of the same slice ten minutes after withdrawal of TNC and TE.

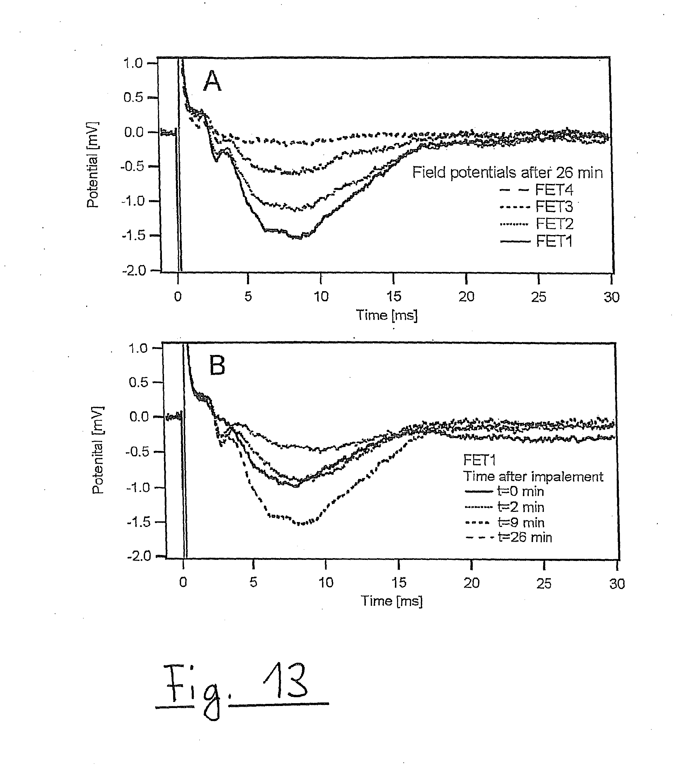

- Fig. 13: shows transistor recordings of field potentials in stratum radiatum of CA1. Fig. 13A shows recordings of four field-effect transistor (FET) interaction sites of a transistor needle chip upon stimulation of the Schaffer collateral 26 min after impalement. After a stimulation artifact and a fiber volley, negative field potentials are recorded due to excitatory synaptic current. The amplitude is high for FET 1 (600µm above the bottom of the slice) and low for FET 4 (840 µm above the bottom). Fig. 13B shows recordings of FET 1 at four different times after impalement. The amplitude decreases within the first minutes and subsequently increases.
- Fig. 14: is a diagram showing the Amplitude of field potential versus time after a first impalement with a TNC in stratum radiatum of CA1. The negative amplitude decreases within the first two minutes and subsequently increases. After withdrawal of the TNC and another impalement at the same place, the enhanced amplitude is attained immediately. Upon another withdrawal and an impalement after an interval of ten minutes, high amplitudes are observed without delay.
- Fig.15: shows profiles of field potential across an acute slice. Three profiles of the amplitudes are plotted for four transistor interaction sites versus the distance from the bottom of the slice. Fig. 15A is a profile during an impalement into stratum pyramidale of CA1 at a place that had been impaled before. Fig. 15B is a profile during subsequent withdrawal. Fig. 15C is a profile during another impalement. The dashed lines mark kinks of the profiles where a transistor interaction sites approaches the surfaces of the slice to the agar layer and to the bath electrolyte.

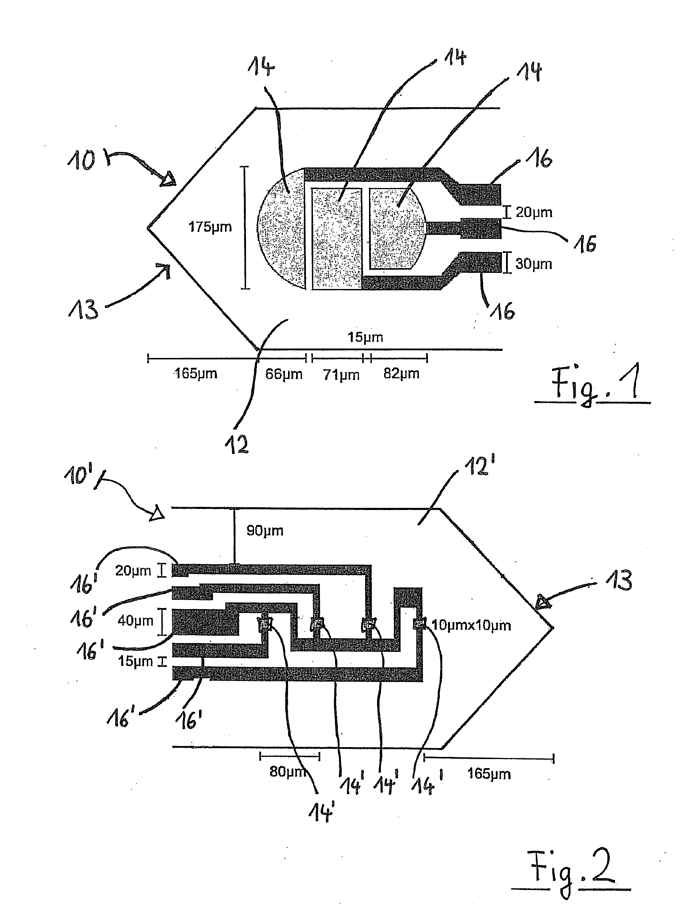

In Fig. 1, a first embodiment of a neurological interfacing probe 10 is shown. The neurological interfacing probe 10 comprises an impalement section 12 having a plurality of interaction sites 14. The interaction sites 14 each are electrically connected by a respective interconnecting trace 16 with a respective contact provided in a contacting section (not shown) of the neurological interfacing probe 10. The neurological interfacing probe is made of silicon. The interaction sites 14 are formed of boron doped P-silicon fields in the impalement section 12. The impalement section 12 further comprises a tip end section 13 having a tip shape, so that the impalement of the impalement section 12 into neuronal tissue is facilitated. The interaction sites 14 are realized as electrodes which are covered by at least one biocompatible insulating layer to provide capacitor functionality for electrically stimulating neuronal tissue into which the neurological interfacing probe 10 can be impaled.

Fig. 2 shows a second embodiment of a neurological interfacing probe 10'. The neurological interfacing probe 10' comprises a impalement section 12' having a plurality of interaction sites 14'. Each interconnecting site 14' is connected by means of two interconnecting traces 16' with two respective contacts provided in a contacting section (not shown) of the neurological interfacing probe 10'. The neurological interfacing probe 10' is made of silicon. The interaction sites 14' are formed by P-silicon fields which make up a respective field effect transistor for each interaction site 14'. The interaction sites 14' show transistor functionality for electrically detecting electrical fields or potentials of neuronal tissue into which the neurological interfacing probe 10' can be impaled.

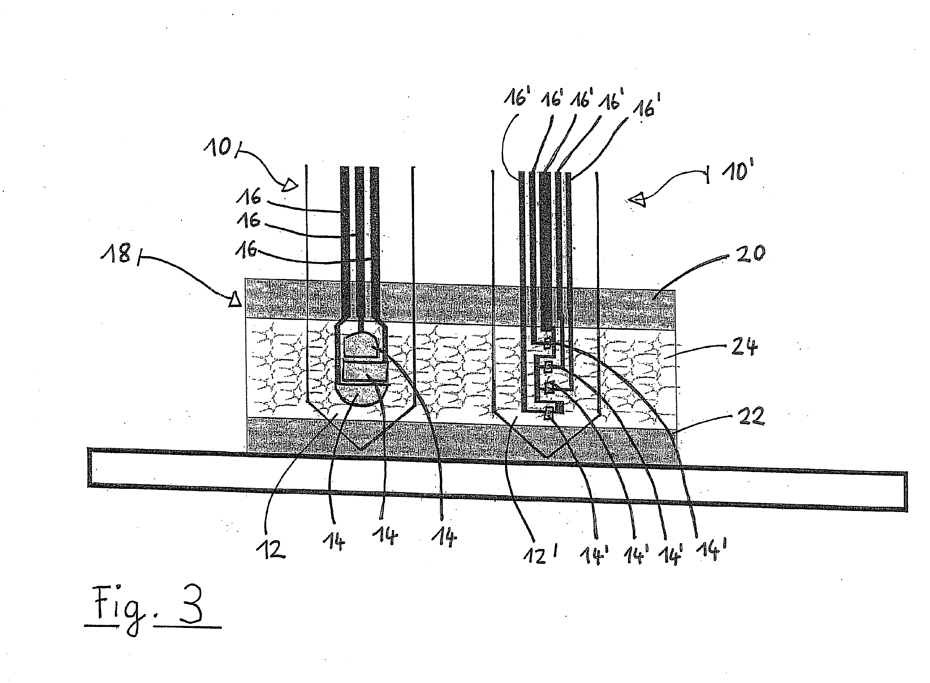

Fig. 3 shows a setup in which the neurological interfacing probes according to the first and second embodiment 10, 10' are impaled into neuronal tissue 18 for stimulation of action potentials (probe 10) and detection (recording) of action potentials (probe 10'). In particular, the neuronal tissue 18 consists of upper and lower layers 20, 22 formed by dead cells and an intermediate layer 24 formed by living cells. After impalement of the neurological interfacing probes 10, 10' into the neuronal tissue 18, the impalement sections 12, 12' are arranged such that the interaction sites 14, 14' are in contact with the living cells of the intermediate layer 24. By means of the neurological interfacing probe 10 having capacitor functionality, the living cells in the intermediate layer 24 can be stimulated. By such a stimulation, action potentials can be excited which in turn can be measured by means of the neurological interfacing probe 10' having transistor functionality.

A further embodiment of the neurological interfacing probe has at least one interaction site which provides capacitor functionality for electrically stimulating neuronal tissue and at least one interaction site providing transistor functionality for electrically detecting electrical fields or potentials of neuronal tissue.

In the following, an embodiment of a manufacturing method for manufacturing a neurological interfacing probe according to the present invention is summarized. The manufacturing method is exemplified with regard to the manufacturing of a neurological interfacing probe having interaction sites providing transistor functionality as well as having interaction sites providing capacitor functionality. Of course, the manufacturing method can easily be adapted for providing a neurological interfacing probe having only interaction sites providing transistor functionality or for providing a neurological interfacing probe having only interaction sites providing capacitor functionality.

The manufacturing of the neurological interfacing probe can be divided into several steps. In a first preparation step, Silicon-On-Insulator (SOI) wafers having an intermediate etch stop layer are labelled by forming labelling marks on the front surface of the wafers. To prepare for the implantation of dopants, oxidation of and structuring of the such formed oxide is carried out. By means of the such structured oxide, the required partial shielding of the wafer for the following implanting step is ensured. By the implantation of the dopants, the basic structures of transistors and capacitors are formed. In a next step, etching is performed for structuring the silicon wafer such that the neurological interfacing probe is obtained.

In the following, the processing of the neurological interfacing probe is laid out in more detail. Afterwards, a precise description of the single processing steps will be given.

First of all, the silicon wafers which serve as substrate for the neurological interfacing probe are cleaned in a complete RCA cleaning step, in order to remove a possible contamination caused during transport and labelling and in order to prepare the wafers for the following high-temperature processing step. The in such a way formed oxide serves to partially shield the wafer from boron ions during the following implantation. The thickness of such an protective oxide is about 600 nm, wherein the protective oxide is formed in a centrotherm furnace. On the such oxidized wafer, by means of lithographic techniques, a photoresist pattern ("adjustment labels") is structured and etched in an 12,5% ammonium fluoride solution for 10 minutes, in order to subsequently etch the oxide layer.

Afterwards, the disposed silicon is etched by means of plasma etching. In this way, recesses are formed on the silicon surface which serve as adjustment labels as well as for numbering of a plurality of chips of a single wafer. The photoresist is removed by means of a CARO bath (duration 10 minutes).

In the following step, during the application of lithographic techniques using an "implantation mask", a tempering step is carried out. Consequently, the wafer is heated to a temperature of about 1000°C for 10 seconds in an RTP furnace and is further covered with photoresist immediately after cool down. After etching the oxide above the interconnecting traces by means of the "implantation mask", the wafers are cleaned again by means of an RCA cleaning step, and a scattering oxide (thickness 23 nm) is created by means of the RTP furnace in the sections of the wafer which are to be implanted with dopants.

For implantation, the wafer is doped by means of 50 keV boron ions at a dose of 5*10¹⁵ atoms per cm².

In a subsequent step, both the mask and the scattering oxide are removed again in an etching step. In a next step, the wafer is cleaned by means of an RCA cleaning step.

In order to ensure electrical isolation and to avoid capacitive effects between the interconnecting traces and the electrolyte into which neuronal tissue is embedded, an insulating layer (a so-called field oxide) can be used. In a first step, a protective oxide having a thickness of 9 nm is formed by means of an RTP furnace, on which in turn an insulating layer having a thickness of approx. 1,4µm is formed by means of PE-CVD.

In the following lithographic step, tempering of the wafer at an increased temperature is carried out , in order to increase the density of the CVD oxide.

In a next step, the surface forming the gate areas is etched. Subsequently, by means of a cleaning step, the photoresist is removed and the formed gate areas are cleaned.

In a subsequent step, a gate oxide having a thickness of 9 nm is created by means of RTO in an area around the gate surfaces. In order to increase the quality of the Si/SiO₂ boundary surface, the wafers are tempered at a temperature of 490°C for 2 minutes in an hydrogen atmosphere.

Next, for formation of capacitive structures, the corresponding photoresist mask is created and the exposed capacitive surfaces are removed from the oxide.

In a subsequent step, an insulating TiO₂ layer having a thickness of 13 nm is formed by means of an ALD method.

In a last step of the manufacturing of the electric structures, metallic bonding pads are formed. After performing a lithographic step by application of a photolithographic "metallization mask", the insulating TiO₂ layer is removed by means of plasma etching, and subsequently the oxide is etched by means of a buffered HF acid.

In a next step, a new photolithographic mask is processed before sputtering the wafer with an aluminium alloy. By means of a lift off process, both the photoresist and the dispensable metallization are removed.

Etching for formation of the outer structure of the neurological interfacing probe starts with etching of the back side of the wafer. In a first step, a mask oxide is created by means of CVD. In order to protect the front side of the wafer which has already been processed as described above, this side is protected by means of a polyimide layer so that contamination is avoided. Subsequently, on the back side an 1,4µm oxide is deposited, and structures by means of the structuring mask "back side" are formed. After removal of the photoresist by acetone, the wafer is mounted in a wafer holder by which the front side of the wafer is protected mechanically, in particular for avoiding etching of the front side. By means of an EDP mask, the backside of the wafer is etched down to the intermediate etch stop layer at a temperature of approx. 95°C, and subsequently the protective polyimide layer is removed again from the front side.

For etching of the front side, a double layer of polyimide is formed. The TiO₂ layer is removed by means of plasma etching, and the silicon dioxide is removed by means of a buffered HF acid so that the silicon surfaces to be etched are exposed. Alternatively, the silicon oxide layer is removed by means of plasma etching as well. In this way, an undesired underetching of the polyimide by means of the buffered HF acid can be avoided.

In order to compensate for an inhomogeneous etching speed, the wafer can be cut into several small pieces, so that plasma etching at the front side can be performed due to the small cut pieces.

In a next step, the 100µm silicon layer is etched in the plasma etching device down to the oxide intermediate layer.

In a subsequent etching step, for example by means of HF or — alternatively
— by means of plasma etching, or by mechanical cutting, the excessive oxide is removed.

In a last step, the remaining polyimide is removed.

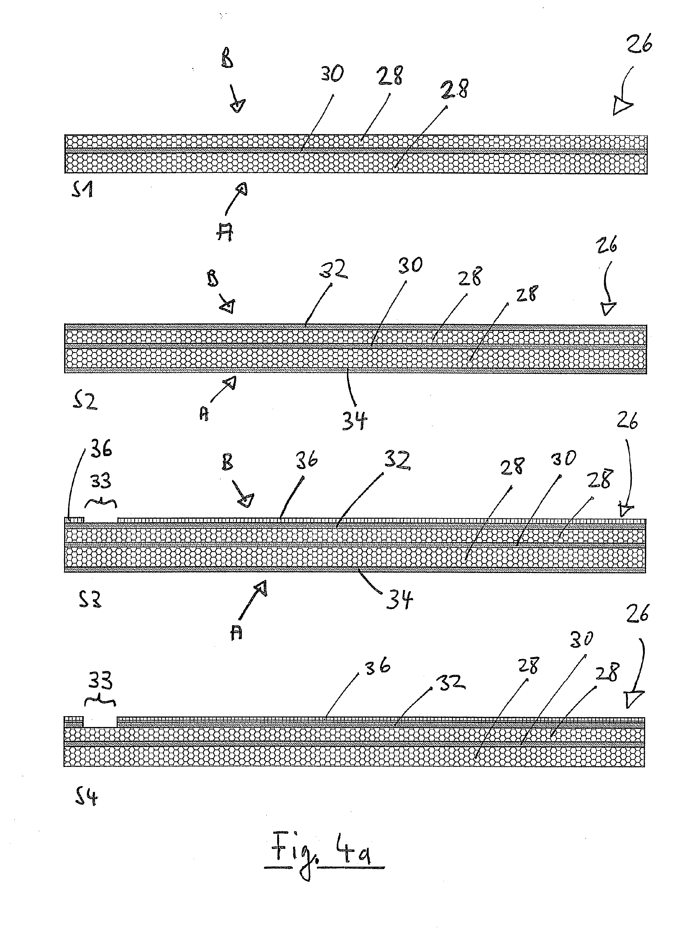

Figs. 4a to 4k illustrate the manufacturing steps S1 to S43 of the above embodiment of the manufacturing method for manufacturing a neurological interfacing probe according to the present invention in detail.

In step S1, a Silicon-on-Insulator (SOI) based semiconductor substrate 26 is provided having a silicon oxide layer 30 serving as an etch-stop layer and being sandwiched in between two n-silicon layers 28. The thickness of the silicon oxide layer 30 is approx. 2µm, the thickness of the SOI substrate is approx. 100µm. Cleaning of the substrate is carried out by using standard methods, for example including the application of a Piranha solution, also known as Piranha etch, e.g. for 15 seconds and the application of an RCA clean for 10 minutes.

In step S2, two silicon oxide layers 32, 34 are formed on top of a first (A) and second (B) side of the semiconductor substrate 26. Oxidation is carried out in a furnace at 1000°C for 3 hours at 1,7 slm O₂ and 3,0 slm H₂. The resulting thickness of the oxide is approx. 680 nm.

In step S3, a photoresist layer 36 is formed on the second side B of the SOI substrate by means of spin coating. The photoresist is soft baken at 90°C for 25 minutes and exposed to light at 21mW/cm² for 5 seconds. Development time using a mx-D-351 developer is 30 seconds. By means of photoresist layer 36, an adjustment label (i.e. a section 33 in which the photoresist layer 36 is removed again using lithographic techniques) is provided for etching.

In step S4, section 33 of the silicon oxide layer 32 in which no photoresist layer 36 is formed is etched away using an 12,5% ammonium fluoride etching mixture (etching duration 12 minutes).

In step S5, the n-silicon layer 28 is partially etched away in section 33 which is not covered by photoresist 36, by application of a plasma etching process (duration 1 minute, 12 sccm SF₆, 10 sccm O₂).

In step S6, photoresist layer 36 is etched away by application of a Piranha solution for 15 seconds.

In step S7, a photoresist layer 38 is partially formed on the second side B of the semiconductor substrate 26 in an analogous way as described in step S3.

In step S8, the silicon oxide layer 32 is etched away in sections 39 which are not covered by the photoresist layer 38 by application of a 12,5% ammonium fluoride etching mixture (etching duration 12 minutes).

In step S9, the photoresist layer 38 is etched away again by application of a Piranha solution.

In step S10, a scattering oxide layer 39 having a thickness of 20 nm is formed in an RTP furnace (at 1130°C, 3 slm O₂, duration 40 seconds).

In step S11, boron atoms (5*10¹⁵ boron atoms/cm²) are implanted at an energy of about 50 keV in sections 40 of the n-silicon layer 28 which are not covered by silicon oxide layer 32. Accordingly, p-doped silicon sections 40 are obtained.

In step S12, silicon oxide layer 32 and scattering oxide layer 39 are etched away using an 12,5% ammonium fluoride etching mixture (etching duration 12 minutes).

In step S13, a protective oxide layer 42 is formed on top of the second side B of the semiconductor substrate 26 in an RTP furnace at 1130°C, 3 slm O₂ (duration 23 seconds, resulting thickness 11 nm).

In step S14, a field oxide layer 44 is formed on top of the second side B of the semiconductor structure 26 by application of PE-CVD (duration 30 minutes, 170 slm SiH₄/N₂ (2%), 720 slm N₂O, 200W, 300°C, 1000 mTorr).

A cleaning process is carried out by application of a Piranha solution for 15 seconds.

In step S15, a photoresist layer 46 is formed on top of the second side B of the semiconductor substrate 26 such that a section 48 remains free of photoresist layer 46.

In step S16, section 48 of the field oxide layer 44 which is not covered by the photoresist layer 46 is etched away using an 12,5% ammonium fluoride etching mixture (etching duration 12 minutes).

In step S17, photoresist layer 46 is etched away using a Piranha solution for 15 seconds.

In step S18, a gate oxide section 50 is formed on top of section 48 of the field oxide layer 44 using a RTP furnace (1130°C, 3 slm O₂, duration 23 seconds, resulting layer thickness 11 nm). The gate oxide section 50 covers a channel section 90 between a source section 92 and a drain section 94 of a field effect transistor 14' which has been formed by the foregoing processing steps.

In step S19, a photoresist layer 52 is formed on top of the second side B of the semiconductor structure 26 in an analogous way as described in step S3 such that a section 54 of the semiconductor structure 26 is not covered by photoresist layer 52.

In step S20, the field oxide layer 44 is partially etched away in section 54 of the semiconductor substrate 26 which is not covered by photoresist layer 52. Etching is performed using an 12,5% ammonium-fluoride etching mixture (etching duration 12 minutes).

In step S21, photoresist layer 52 is etched away by application of a Piranha solution for 15 seconds.

In step S22, an insulating TiO₂-layer 56 is formed (13 nm layer thickness) on top of the second side B of the semiconductor substrate 26. A portion 96 of p-doped silicon section 40 covered with the TiO₂-layer serves as a capacitor electrode to provide capacitor functionality.

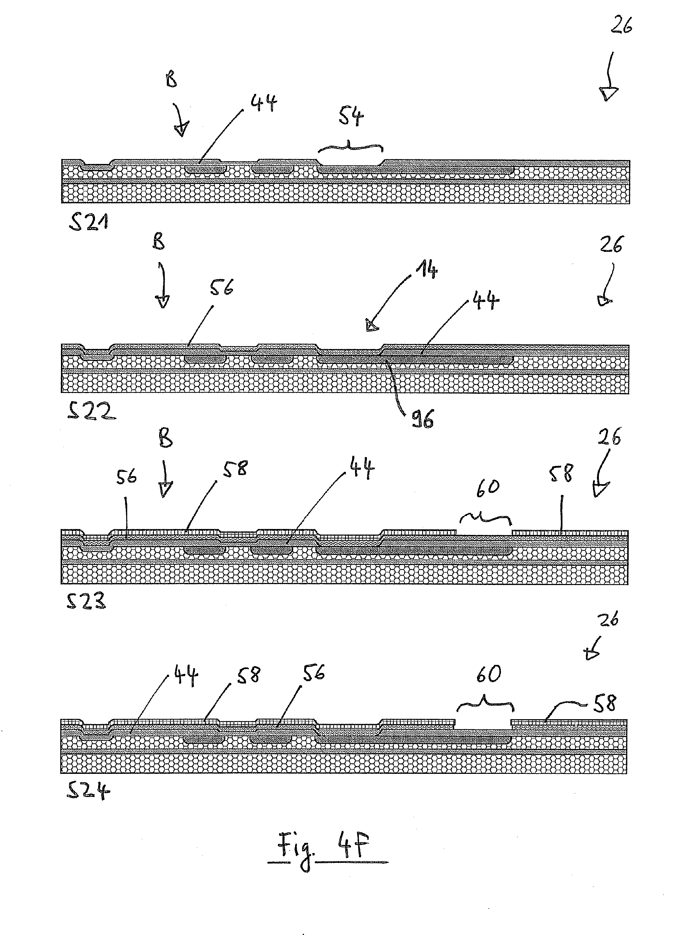

In step S23, a photoresist layer 58 is formed on top of the second side B of the semiconductor substrate 26 except for sections 60 (only one section is shown in Fig. 4f) of the semiconductor substrate 26. Step S23 is carried out in an analogous way as step S3.

In step S24, the insulating layer 56 is partially etched away in sections 60 which are not covered by photoresist layer 58. For cleaning, plasma etching is performed for 2 minutes (3 sccm O₂, 50 W, 500 mTorr). Etching of the insulating layer itself is performed using 30 sccm CHF₃ for 14 minutes (at 30 sccm Ar 50 W, 50 mTorr). For cleaning, 30 sccm O₂ is applied for 2 minutes (50 W, 500 mTorr).

In step S25, the field oxide layer 44 is etched away in sections 60 which are not covered by photoresist 58. For etching, an 12,5% ammonium fluoride etching mixture is applied for 12 minutes.

In step S26, the semiconductor substrate 26 is cleaned by application of a

Piranha solution. Subsequently, an AlSi alloy 62 having a thickness of 150 nm is sputtered on top of the second side B of the semiconductor structure 26.

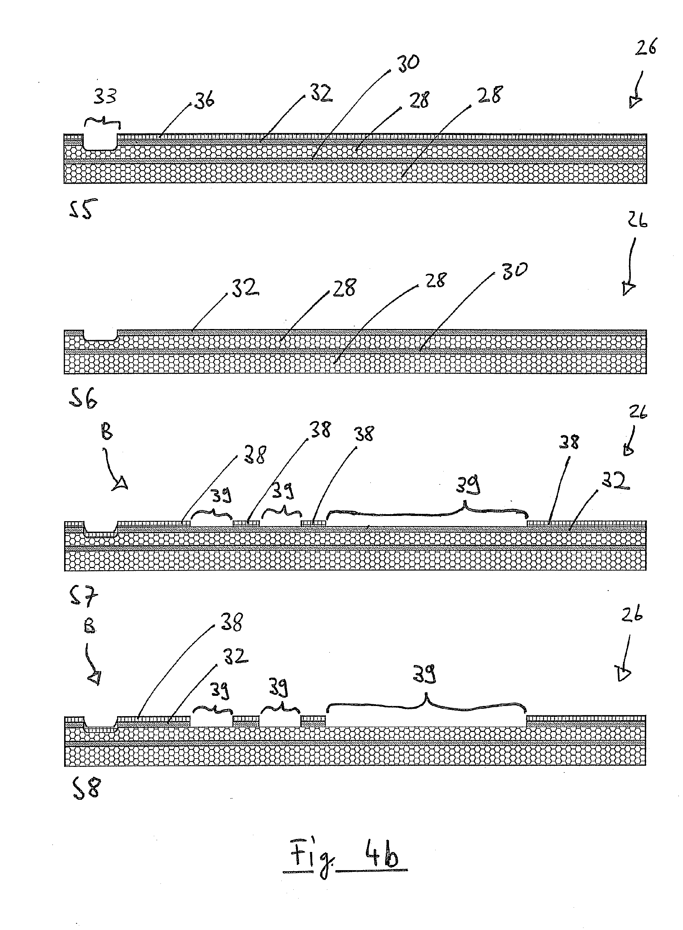

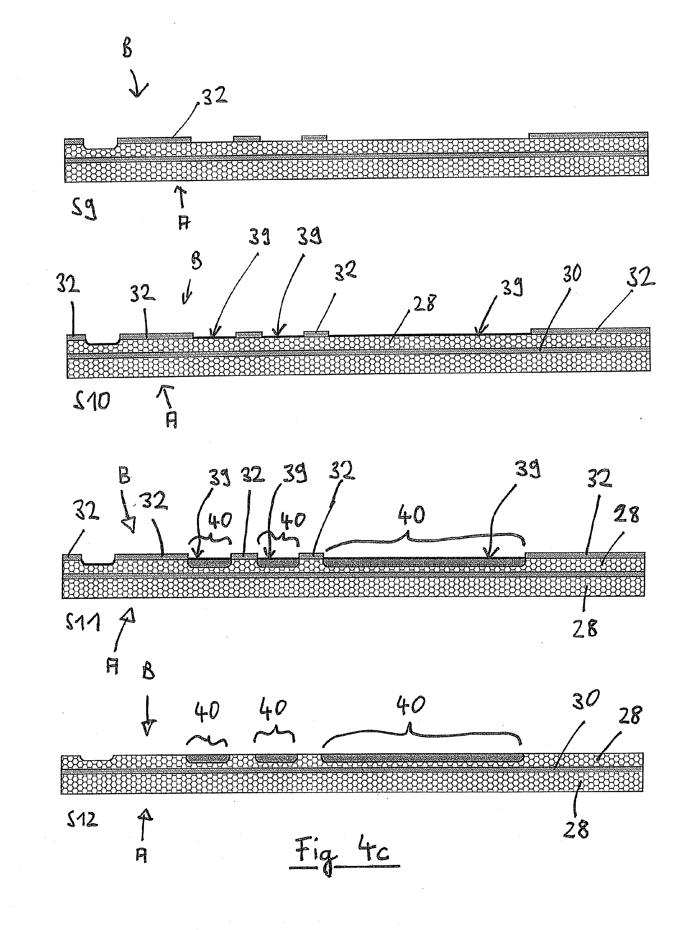

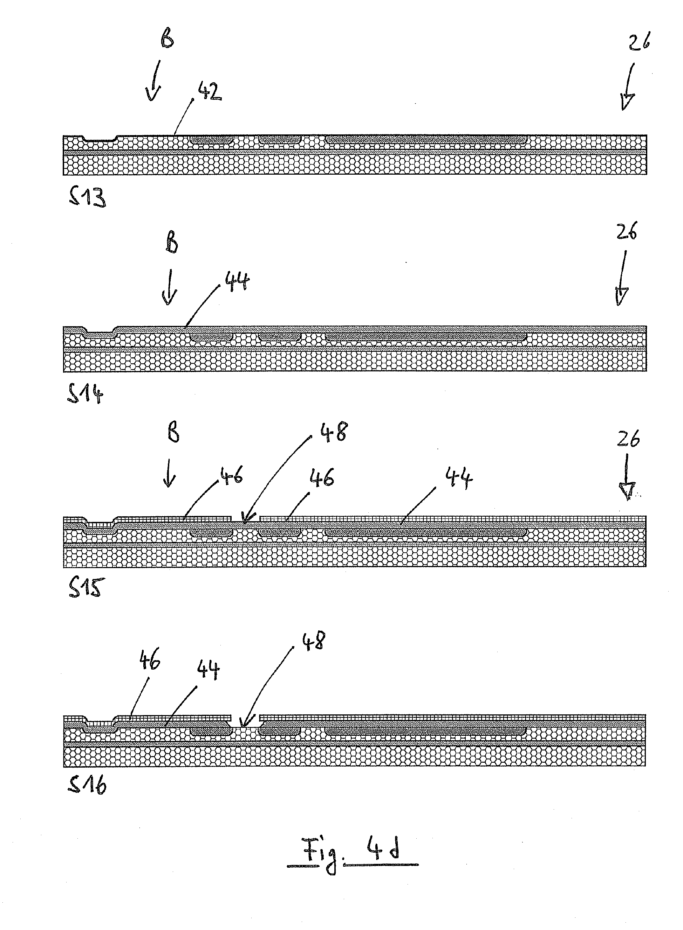

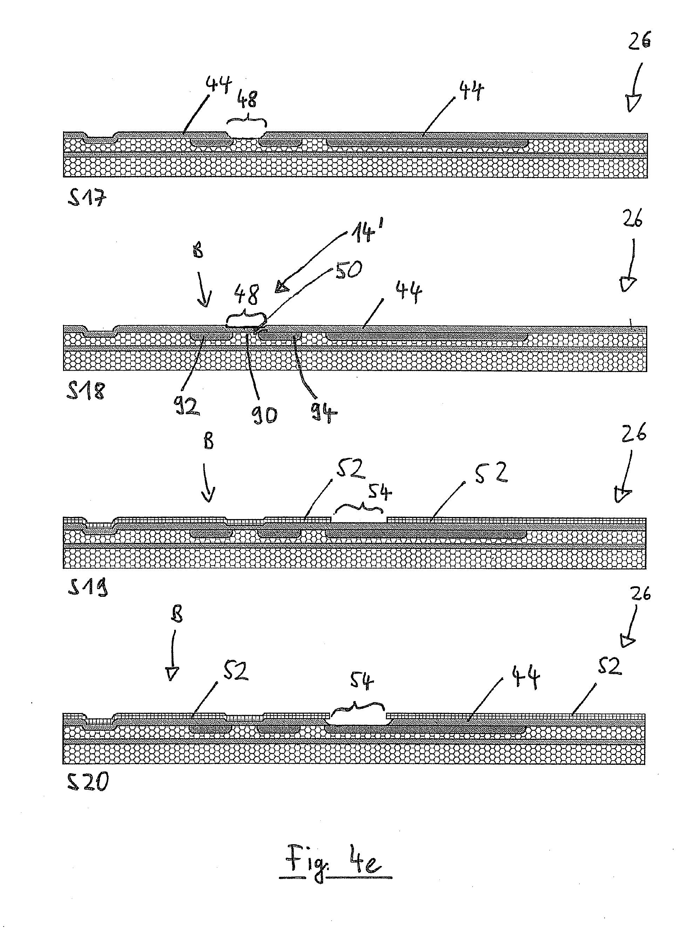

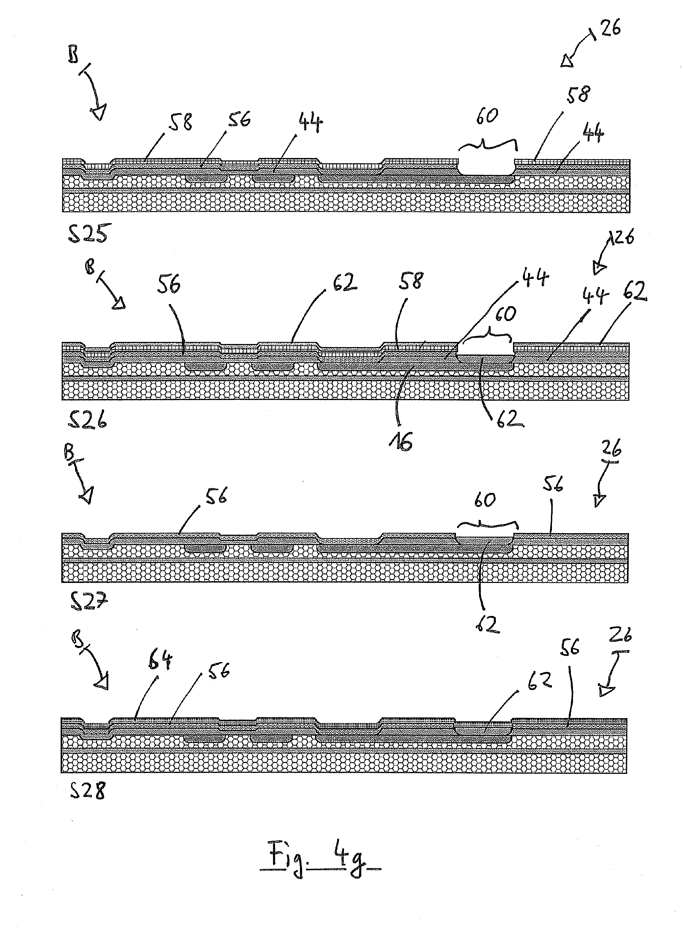

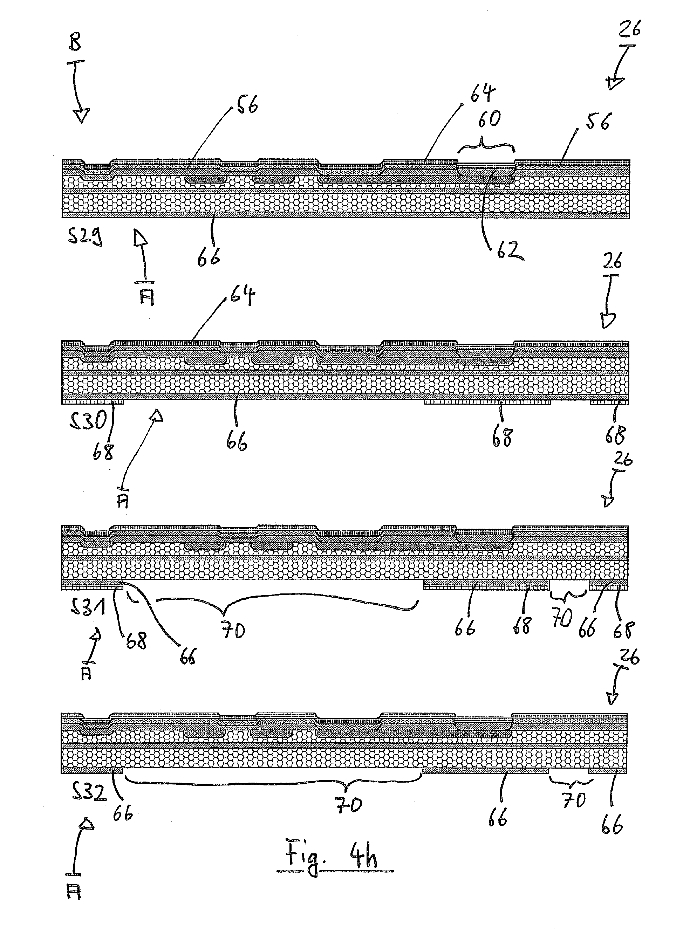

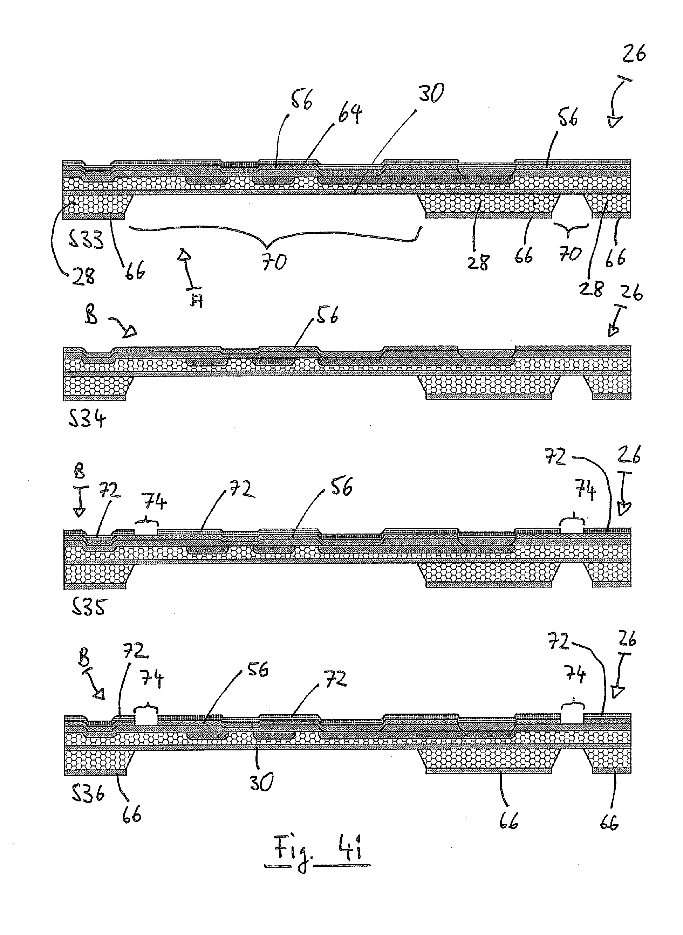

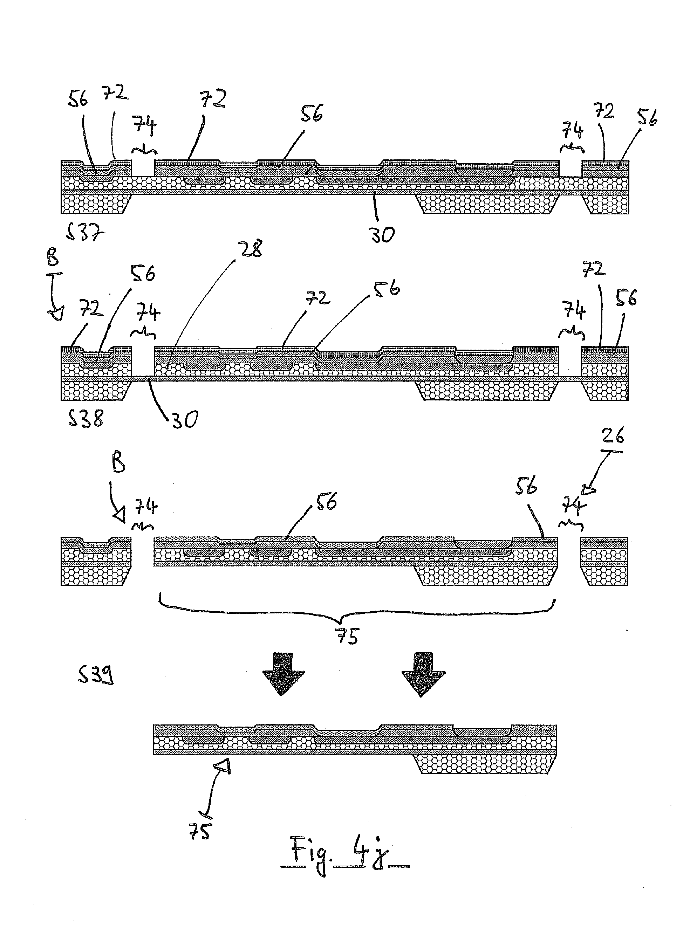

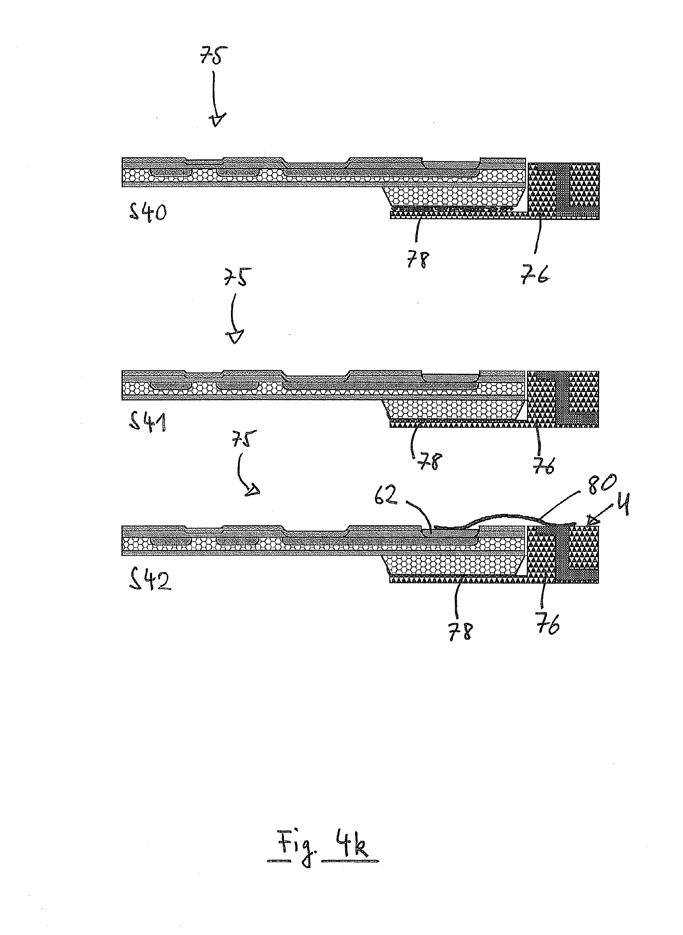

In step S27, a lift off process is performed in a furnace at 110°C and in an acetone ultrasonic bath. After completion of the lift off process the AlSi alloy is removed from the second side B except for the sections 60 where the AlSi alloy layer forms a respective contact field. For each capacitor electrode 96 of an interaction site 14 there is a respective contact field 62, which is connected with the respective electrode 96 by means of an interconnecting trace 16 which is a portion of a respective p-doped silicon section 40. Similarly, the source section 92 and the drain section 94 of a respective field effect transistor 14' each are connected by respective interconnecting traces formed by a portion of a respective p-doped silicon section 40 with a respective contact field (not shown in Fig. 4).

In step S28, a polyimide layer 64 is formed on top of the second side B of the semiconductor substrate 26 by means of spin coating. The polyimide layer 64 is heated in a furnace for 30 minutes at 100°C followed by heating for 30 minutes at 200°C, followed by heating for 60 minutes at 300°.

In step S29, a silicon oxide layer 66 is formed on the first side A of the semiconductor substrate 26. The silicon oxide layer 66 is formed in an analogous way as described in step S13.

In step S30, a photoresist layer 68 is partially formed on the first side A of the semiconductor substrate 26 in an analogous way as described in step S3.

In step S31, the silicon oxide layer 66 is etched away in a section 70 of the semiconductor substrate 26 which is not covered by photoresist layer 68.

In step S32, the photoresist layer 68 is removed by acetone.

In step S33, the boron doped silicon layer 28 is etched away on the first side A of the semiconductor substrate 26 in section 70 which is not covered by silicon oxide layer 66. Etching is performed using an EDP-"S" mixture for a duration of 8 to 16 hours at a temperature of 95°C.

In step S34, the polyimide layer 64 is removed from the second side B of the semiconductor substrate 26 by application of the so-called micro strip 2001 method (50% N-methyl pyrrolidon, 50% 2-(2-aminoethoxy)-ethanol).

In step S35, a polyimide layer 72 is formed on the second side B of the semiconductor substrate 26 except for sections 74. The sections 74 are formed using standard photolithographic techniques.

In step S36, the insulating layer 56 is etched away in section 74 which is not covered by polyimide layer 72 on the second side B of the semiconductor substrate 26. Etching is carried out in an analogous way as described in step S24. The first side A of the semiconductor substrate 26 is protected from being etched by mechanical means, namely a substrate holder. The same applies for all subsequent etching steps directed to the second side B of the semiconductor substrate 26 only.

In step S37, the silicon oxide layer 66 is etched away by application of a 12,5% ammonium fluoride etching mixture (duration 12 minutes).

In step S38, the phosphor doped silicon layer 28 is etched away on the second side B of the semiconductor substrate 26 in sections 74 which are not covered by polyimide layer 72. For etching, an etching cycle having alternating etch and passivation steps, for example a kind of modified Bosch Cycle, is applied (step A: 50 sccm SF₆ for 30 seconds at 50 mTorr, 100 W; step B: 50 sccm CHF₃, 100 mTorr, 100 W for 60 seconds). Referring again to step S37, the silicon oxide layer 66 may alternatively etched away by application of the modified Bosch cycle as described in step S38. For further handling it may be appropriate to maintain narrow silicon oxide layer ridges between the respective sections to be used as neurological interfacing probes, which can later be broken.

In two following steps, identified in Fig. 4j as S39, the silicon oxide layer 30 is etched away using an 12,5% ammonium fluoride etching mixture, and the polyimide layer 72 is removed using a microstrip 2001 solution. Alternatively, the silicon oxide layer can be removed using a plasma etching step. Afterwards, in addition or alternatively to step S22, a protective polyimide layer (not shown in the figures) is formed on the second side B of the semiconductor substrate 26.

Thereafter, a portion 75 or portions 75 of the semiconductor substrate 26 is/are separated and thereby obtained by mechanically braking the semiconductor substrate 26 in close proximity to sections 74. Alternatively, an etching process (e.g. by application of a 12,5% ammonium fluoride etching mixture) could be used for separating a portion 75 or, preferably, a plurality of portions 75 of the semiconductor structures 26 which shall serve as a neurological interfacing probe.

In step S40, such a semiconductor structure portion 75 obtained from the preceding processing step is placed on a printed circuit board 76 by means of a bonding wax 78.

In step S41, the semiconductor structure portion 75 is fixed on the printed circuit board 76 by heating the bonding wax 78 in a furnace at a temperature of 110°C.

In step S42, respective end sections of elongated metal contacts 80 are mounted both on a respective AlSi layer 62 and an upper contacting section U of the printed circuit board 76. Accordingly, it is possible to electrically connect with the source section 92 and the drain section 94 of each interaction site 14' and to electrically connect with each respective capacitor electrode 96 of each interaction site 14 by means of a respective contacting section U, a respective metal contact 80 and respective interconnecting traces formed by p-doped silicon obtained from the implantation step.

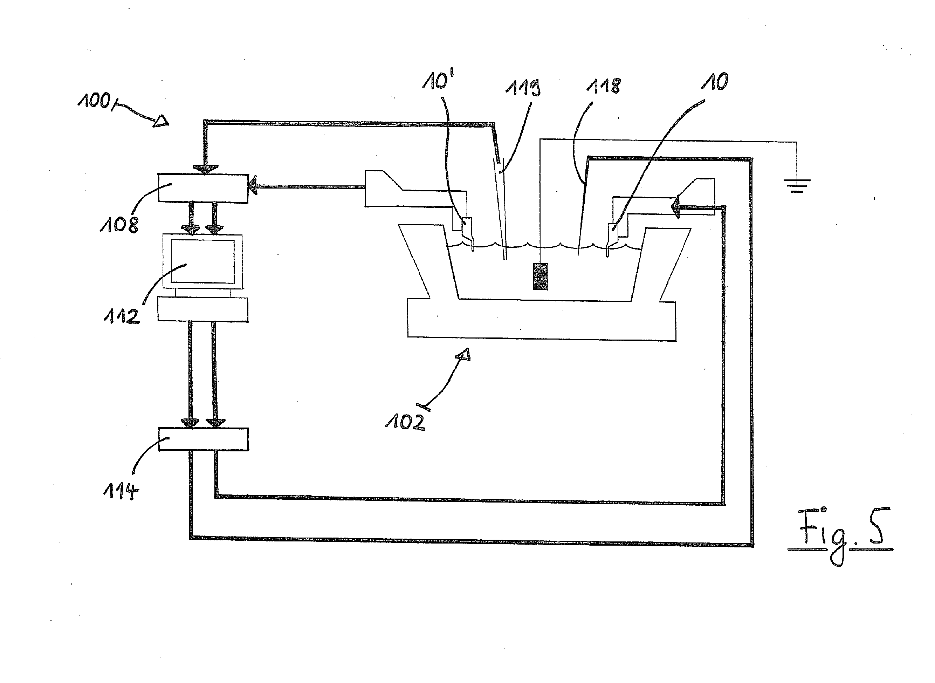

In Fig. 5, a measurement setup 100 for demonstrating successful coupling between slices of a brain and the neurological interfacing probe according to the present invention is shown. The measurement setup 100 comprises a medium chamber 102 in which the brain slice to be analysed is positioned. The setup 100 further comprises a neurological interfacing probe for stimulation 10 (having capacitor functionality) and for recording 10' (having transistor functionality) of electrical signals and therefore of neuronal activity in the brain slice. For stimulating and recording the respective neurological interfacing probe can be impaled into the brain slice as shown schematically in figure 3.

The neurological interfacing probe for recording 10' is connected with an amplification unit 108 which amplifies detected electrical signals which correspond to respective action potentials. The amplification unit 108 is connected to a computer 112 to which the amplified signals are transferred for further processing and displaying. The neurological interfacing probe for stimulating 10 is connected to a control unit 114 which in turn is connected to computer 112. By means of control unit 114 electrical signals are supplied to the neurological interfacing probe 10 for stimulation of the brain slice. The connection between the respective neurological interfacing probe and the amplification unit 108 and the control unit 114, respectively, is effected by appropriate wiring and connectors which connect with the terminals of the respective contacting section of the respective neurological interfacing probe, so that a connection with the respective interaction sites is obtained.

For test purposes, an action potential in the brain slices can alternatively be stimulated and recorded by means of a tungsten electrode 118 and a glass electrode 119, respectively. The tungsten electrode 118 is connected to the control unit 114 for supplying controlled current pulses into the brain slice. The glass electrode 119 is connected to the amplification unit 108 for amplification of detected electrical signals.

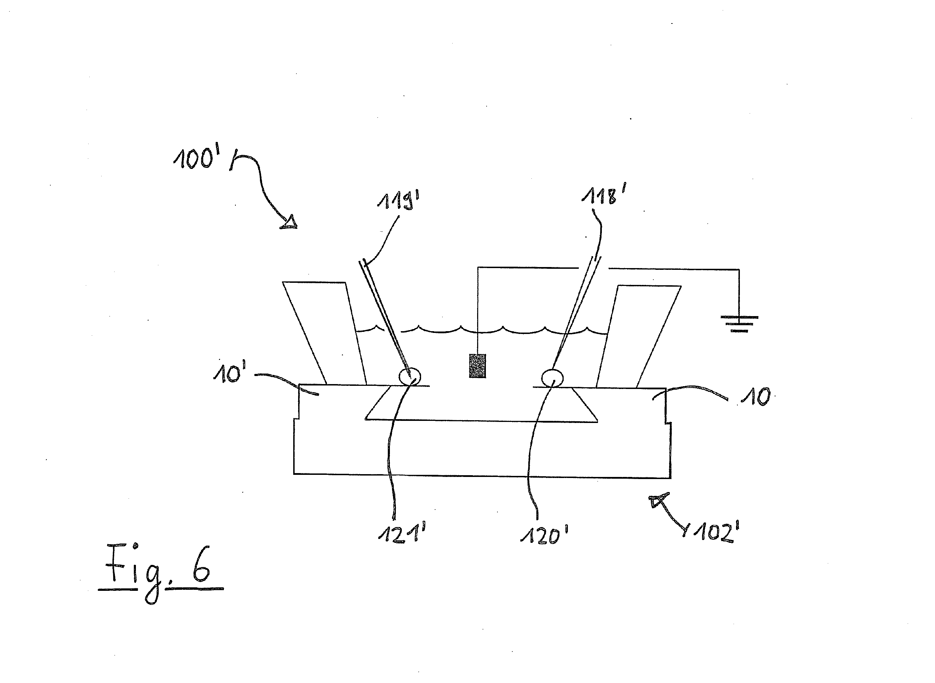

In figure 6, a modified setup 100' for measuring single neurons 120', 121' instead of brain slices is shown. The major components of the modified setup 100' correspond to the setup 100 described above except that in the modified setup 100', the neurological interfacing probes for stimulation and recording 10, 10' are not impaled into neurological tissue, but are rather used as planar sensors.

The functioning of the neurological interfacing probe for recording and of the neurological interfacing probe for stimulating was demonstrated using the setup according to Fig. 6 for measurements effected on separated neurons and using the setup according to Fig. 5 for measurements effected on brain slices.

For the measurements with the setup according to Fig. 6 neurons of the lymnaea stagnalis were used, which are shown schematically in Fig. 6 at 120' and 121'.

The cochleas of said snails were separated from their body, intoxicated and brought into a sterile environment for further processing. In order to set the neurons free, the animal was cut from the back side, and the central nervous system was isolated. The nervous system was then separated into several ganglia. In such ganglia, the cell bodies of the neurons are provided on the surface. The axons of the neurons are directed inwardly and do provide network functionality. In order to separate the neurons from the tissue, an enzyme treatment was carried out using trypsin for a duration of 23 minutes. A following inhabitation step deactivated the trypsin thereby ensuring that only inter-cellular connections were treated.

Next, a skin-like layer of tissue which envelops the ganglia was carefully removed by means of a pair of tweezers. Single neurons were taken up by means of a glass pipette. Single neurons 120', 121' were then positioned on the neurological interfacing probes 10, 10' as shown in Fig. 6 after effecting an additional coating step (using laminin fragment YIGSR) for improving the adhesion of the cells on the neurological interfacing probes 10, 10'.

Before starting the electrical measurements, the neurons 120', 121' were kept immobile for at least one hour in order to ensure an optimized adhesion. For initiation of the measurements, the electrodes 118', 119' were impaled into the single neuron 120', 121', respectively, and the vitality of the neurons 120', 121' was tested by activation of an action potential using the electrodes 118', 119' as described above. In particular, for recording of action potentials, both electrodes 118', 119' were impaled into single neuron 121'. By means of electrode 118', a current pulse was injected into the single neuron 121', thereby exciting an action potential. The action potential was then recorded both by electrode 119' and the neurological interfacing probe for recording 10'.

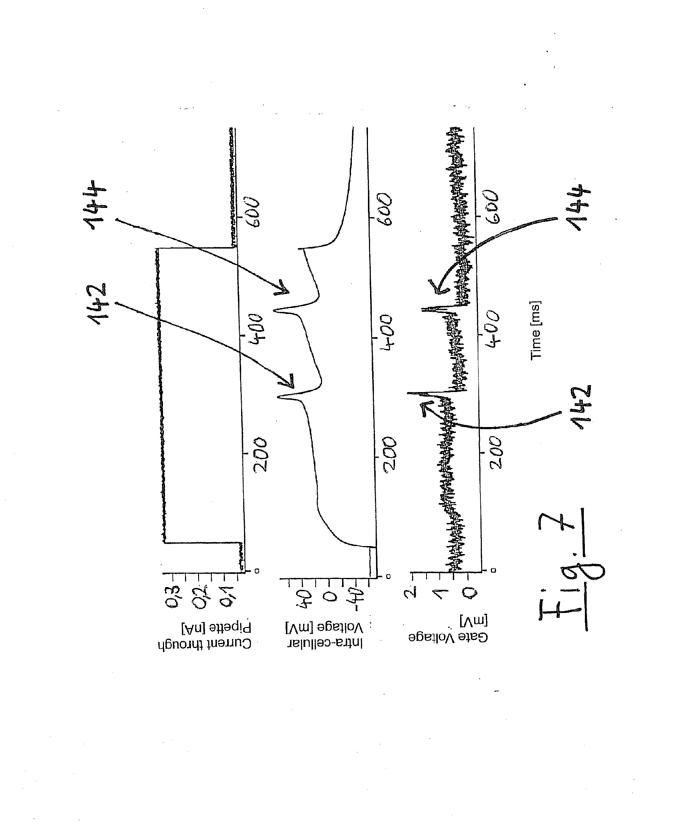

Diagram (a) of figure 7 shows a current pulse flowing through tungsten electrode 118' in order to excite action potentials in the single neuron 121'. Diagram (b) shows the excited action potentials as measured by means of glass electrode 119', while diagram (c) shows the same excited action potentials as measured by means of the neurological interfacing probe 10' having transistor functionality. In the measured intra-cellular values of diagram (b) an offset voltage of about -60 mV corresponding to the steady state potential of the snail cells can be identified. While this action potential is being excited, a potential difference of up to 1.5 mV is detected by means of the neurological interfacing probe 10'. These positive signals are caused by the flow of potassium ions wherein two positive voltage peaks 142, 144 are detected. During the depolarisation of the cell membrane by means of the flow of sodium ions at the free cell membrane, due to the modified cell membrane voltage, an increased flow of potassium ions at the adhered membrane is observed, leading to the first peak 142. The second peak 144 is caused by potassium ions as well which pass through the opened ion channels.

For testing the functionality of the neurological interfacing probe 10 having capacitor functionality, the glass electrode 118' was impaled into single neuron 120'.

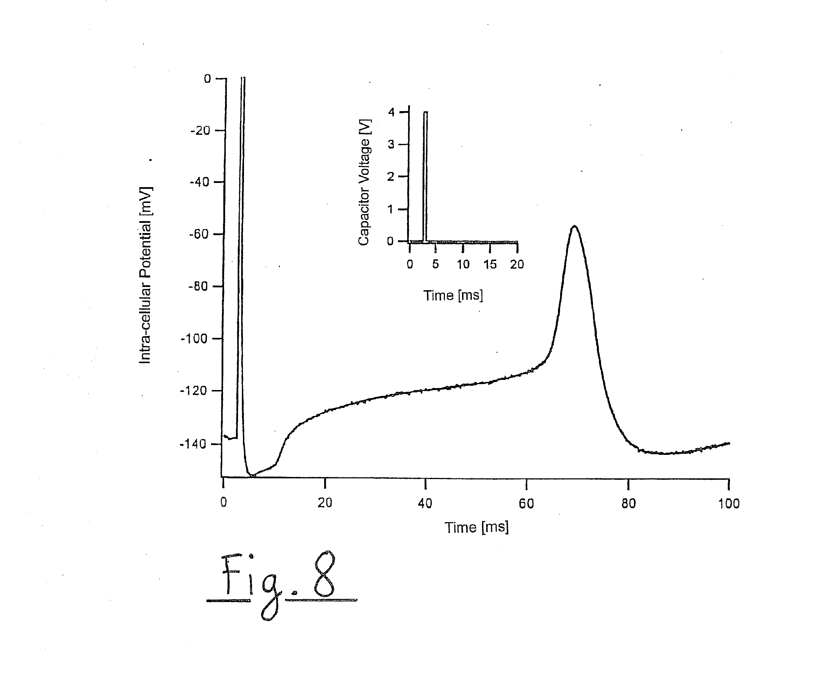

By means of a capacitive electric stimulation an action potential was excited in the single neuron by means of the neurological interfacing probe 10 as shown in figure 8. Thereby, by means of a strong change in the applied voltage, a large voltage gradient 130 was created on the cell membrane so that holes were formed in the cellular membrane. As a consequence, positive ions could infiltrate in the cell through these holes, thereby depolarising the cell and exciting an action potential.

Alternatively, a stimulation by means of ascending and descending ramp voltage signals created in the neurological interfacing probe 10' having capacitor functionality can be used for exciting action potentials in a neuron. Measurements showed indeed that the electric potential of the cell membrane can be increased by means of such voltage ramps. Therefore, by means of a selection of an appropriate combination of duration and slope of the ramp signals, a capacitive stimulation in a single neuron is possible.

For demonstration measurements with the measurement setup 100 of Fig. 5, brain slices of the hippocampus of rats were used. The main function of the hippocampus is to transfer information from the short-term memory to the long-term memory of the brain. The hippocampus has a lamella-like structure, so that neuronal activity takes place within a specific layer of the brain structure. This makes it possible to cut specific layers which are neuronally active over several hours under optimized conditions. In order to obtain brain slices suitable for action potential measurements to be carried out with measurement setup 100, a 30-day old rat was intoxicated with isoflurane, and so the skullcaps and meninges were removed afterwards. The brain was then removed and put in an ice-cooled culture medium (ACSF: artificial cerebrospinal liquid). Regarding its composition, the culture medium was composed similar to the liquid of the brain and was also additionally provided with carbogen (95% O₂, 5% CO₂). The oxygen contained in carbogen entered into the brain tissue, and the carbon dioxide acted as an pH buffer for the medium. The brain was unravelled carefully in order to set to hippocampi free. Cutting was performed by means of a vibratom which cuts the tissue with its vibrating blades in a gentle way. In order to position the hippocampus correctly on the neurological interfacing probes and to fix thereon, the hippocampi were sticked on mounting pads and stabilised during cutting by means of a so-called agar block. The such obtained cuts having a thickness of about 550 µm were kept in the medium provided with gas for 2 hours for the purpose of regeneration. As the brain slices were cut such that their thickness was very low, a sufficient provision of oxygen and nutrients are ensured, so that the cuts kept alive for several hours.

The stimulation of action potentials was done by means of tungsten electrode 118 which was impaled into the brain slice in a similar way as the neurological interfacing probe 10' having transistor functionality. For recording of stimulated electrical signals, by means of the neurological interfacing probe 10' being impaled into the brain slice, the drain voltage of a respective transistor of the neurological interfacing probe 10' was set to 0.12 V and the source voltage was set to V_{S} = 2.0 V during measurements. A bulk voltage of V_{B} = 5 V was applied to the bulk such that an sufficient isolation of the interconnecting traces was ensured. By means of these settings measurements with the brain slices were performed. By application of a current pulse of 100 µA flowing through the tungsten electrode 118 for 0,1 ms, action potentials in the brain slices were excited, which in turn were measured by means of the neurological interfacing probe 10' using respective interaction sites having transistor functionality thereof.

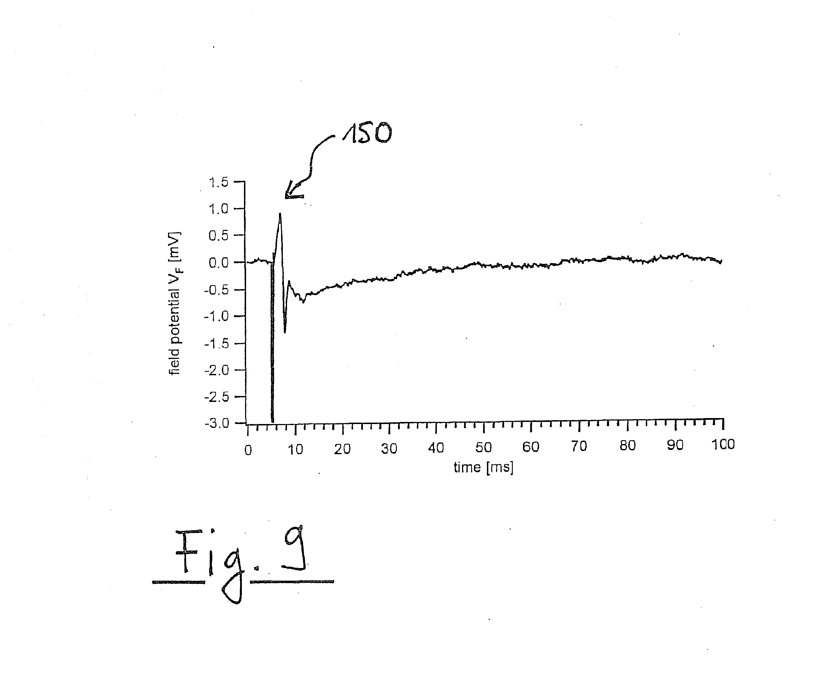

In the diagram of figure 9 showing the field potential of the brain slice as recorded by one of said interaction sites of the neurological interfacing probe for recording 10', electrical pulses 150 excited by the electrode 118 can be clearly identified.

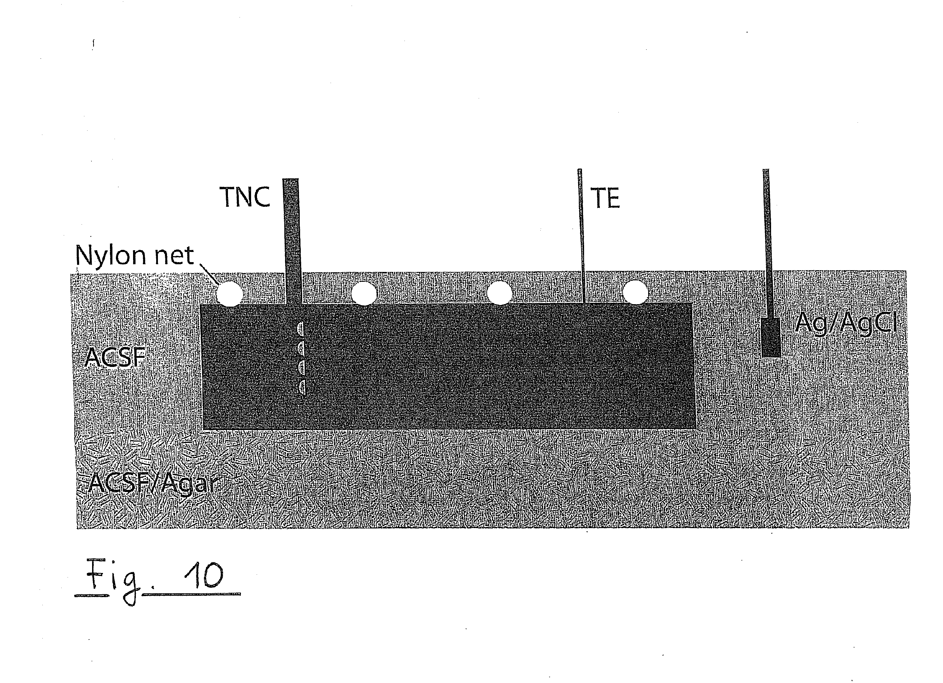

In the following, further results are presented which demonstrate that efficient transistor recording is possible in a geometry that corresponds to the interfacing of brain tissue in a living animal. In the test experiments, neurological interfacing probes made from silicon chips generally were impaled into thick acute slices from rat hippocampus as illustrated in Fig.3. In the test experiments, needle-shaped silicon chips with an inert and biocompatible surface of titanium dioxide were impaled into thick acute slices from rat hippocampus as further illustrated in Fig. 10. Local field potentials were recorded with transistors in stratum radiatum of CA1 when the Schaffer collateral was stimulated. Signals with large amplitudes were observed in the central layer of the slice where the tissue is not damaged by the cutting process. In the following, the details of the test experiments and the underlying materials and methods are described using the language of a scientific paper, mostly using present tense.

### Materials and Methods:

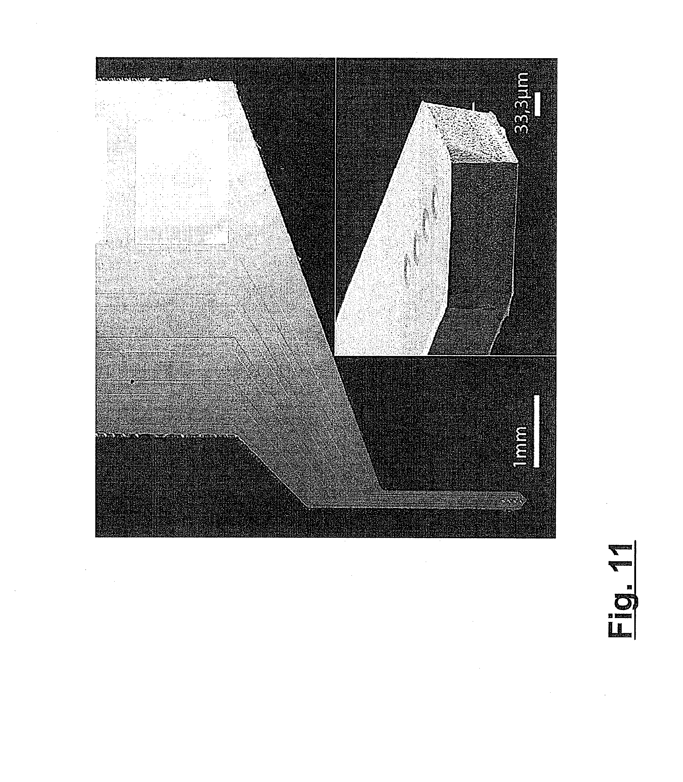

**Chips.** Neurological interfacing probes, which in the following are denoted as transistor needle chips (TNCs), are fabricated from silicon-on-insulator (SOI) wafers (4 inches) with a layer of 100 µm n-type silicon (1-10 Ωcm) on 2 µm SiO₂ with a 400 µm silicon substrate (SiMat, Landsberg/Lech, Germany). A TNC consists of two domains, the needle with transistors and a contact plate with bond pads. The needle is 2 mm long, 360 µm wide and 100 µm thick. An array of four transistors (gate area 10 µm x 10 µm) starts 175 µm from the tip of the needle with a distance of 80 µm between the transistors. The contact plate is 10 mm long, 5 mm wide and 500 µm thick. The axis of the needle is laterally displaced with respect to the contact plate to allow a microscopic control of impalement. A micrograph of the TNC and a scanning electronmicrograph of its tip are shown in Fig. 11.

In a first step of processing, the transistors are fabricated on the top Si layer of the SOI wafer as described for simple Si wafers (Weis et al., 1996; Weis & Fromherz, 1997). In short: a mask oxide (650 nm) was formed by wet thermal oxidation. Source and drain with connecting lanes to the contact plate are opened by wet etching (12.5% buffered fluoric acid), coated with 20 nm scattering oxide by a rapid thermal process and doped with boron by ion implantation (6.5*10¹⁵ cm⁻², 50 keV). Then the mask oxide is removed by fluoric acid. A field oxide (1 µm) is deposited by plasma enhanced chemical vapour deposition (PECVD) (Plasmalab 80+, Oxford Instruments, Abingdon, UK) and circular windows (about 20 µm in diameter) are etched. A gate oxide (11 nm) is created by a rapid thermal process and annealed in hydrogen atmosphere at 490°C. A layer of TiO₂ (13 nm) is deposited by atomic layer deposition (ASM, Helsinki, Finland) to improve the stability and biocompatibility of the chip (Hutzler & Fromherz, 2004). The TiO₂ on the contact areas of source, drain and bulk silicon is removed by plasma etching (RIE Plasmalab 80+) in a 1:1 CHF₃/Ar atmosphere (30/30 sccm, 50 mTorr, 50 W, 14 min) and the SiO₂ by fluoric acid. Finally the contact areas are metallized by sputtering with a Al/Si (6%) alloy (150 nm).

In a second step of processing, the needle and the contact plate are formed by two etching processes (Ensell et al. 2000). Appropriate measures are taken to protect the transistors. On the back of the SOI wafers a mask oxide is made by plasma enhanced chemical vapour deposition. The silicon substrate is removed around the TNCs and under the needles using a solution of ethylene diamine pyrocatechol (Reisman et al., 1979). A special wafer holder (AMMT GmbH, Frankenthal, Germany) is used to protect the front side of the wafers. Subsequently, the silicon of the front side is removed around the TNCs by a plasma process (RIE Plasmalab 80+) with alternating etching and passivation steps using an SF₆/O₂ atmosphere for etching (50 sccm/10 sccm, 50 mTorr, 100 W, 30 s) and a CHF₃ atmosphere for passivating the walls (50 sccm, 100 mTorr, 100 W, 30 s) (Syau, et al., 1991; Sowa et al., 2000). A polyimide layer (32 µm) (PI 2556 HD MicroSystems, Neu-Isenburg, Germany), protects the surface of the TNC. The layer of 2 µm SiO₂ of the SOI wafer remains on the back as an insulation. No additional process is applied to coat the sidewalls. After complete processing, the contact plates are still connected to the wafer by a thin bridge that is broken when the TNCs are used. An individual TNC is glued with its contact plate to a small printed circuit board and wire bonded. The bond contacts are insulated with a silicone adhesive.

More details of a suitable manufacturing method for manufacturing the neurological interfacing probes in the form of transistor needle chips (TNCs) are given above in the context of Figures 1 to 4.

**Slices.** Acute hippocampal slices are prepared by standard techniques (Dingledine, 1984; Korte et al., 1996). Male whistar rats (25 days old) are anaesthetized with isoflurane and decapitated. After quick dissection the brain is placed in ice-cold artificial cerebrospinal fluid (124 mM NaCl, 3 mM KCI, 1.25 mM KH₂PO₄, 2 mM MgSO₄, 26 mM NaHCO₃, 2.5 mM CaCl₂, 10 mM glucose), that is aerated with 95% O₂ / 5% CO₂. The hippocampi are isolated and the dorsal part is cut by a vibratom (VT 1000S, Leica) into 800 µm thick transverse slices. During cutting the hippocampi are immersed in cold artificial cerebrospinal fluid and stabilized by an agar block. Then the slices are kept at room temperature for about 2 hours. For the measurements a culture plate inlet (Millicell, Millipore) is coated with agar (about 1 mm) to prevent breaking the TNCs during impalement. The inlet is inserted into a plexiglass holder and perfused with aerated artificial cerebrospinal fluid. After 15 minutes, the slice is transferred to the dish and held on the agar with a nylon net (mesh size 700µm) using a micromanipulator.

**Electrophysiology.** The slices are stimulated by a monopolar tungsten electrode (AMS 5753; Science Products, Hofheim, Germany) using a stimulus isolator (A360D, WPI, Berlin, Germany). In test experiments, micropipettes (borosilicate glass, 3 M NaCl, chlorinated silver wire) are used to record field potentials (Axoclamp 2B; Axon instruments, Foster City, USA). Free micropipettes are used as well as micropipettes that are mounted with their tips close to the transistors of the TNC. The bath is held at ground potential with two Ag/AgCl electrodes near the inlet and outlet of the artificial cerebrospinal fluid. The signals are low-pass filtered at 1 kHz.

The operating point of the transistors is defined by +0.25 V applied to the sources and to bulk silicon with respect to the bath electrode and by a drain voltage -0.25 V with respect to the source. The source drain current is around 6 µA. This operating point with small currents is chosen for lower noise of the transistors. The chips are connected to a custom build amplifier. Signals are low pass filtered at 3 kHz. Before a measurement, the transistors are calibrated by applying voltage pulses of 1 mV/100 ms to the bath electrode and recording the change of the source-drain current. With the relation δI^{cal}_{D} = gₘ δV^{cal}_{bath} the transconductances in a range of gₘ = - 15pS are obtained. They are used to evaluate the field potentials from changes of the source-drain current in stimulation experiments with ϕ = δI_{D} / gₘ. These signals have been shown to agree with field potentials that are recorded with a micropipette (Stangl & Fromherz, 2008).

**Protocol.** All experiments are performed at room temperature. A TNC and a tungsten electrode are attached to two programmable micromanipulators (5171, Eppendorf, Hamburg, Germany). At first, the TNC is placed in the artificial cerebrospinal fluid and the transistors are calibrated. Then the tungsten electrode is impaled into the Schaffer collaterals and stimulation pulses (100 pA, 100 µs) are applied. In a typical experiment, a TNC is impaled at a speed of 500 µm/s into stratum radiatum of the CA1 region in steps of 400 µm. When the first transistor picks up a large signal, the impalement is stopped and the response of all four transistor is recorded every 5 s for one minute and every 10 s for about 30 min. Then the TNC is withdrawn, and again impaled to the same position with subsequent recordings of the field potential. The TNC is withdrawn a few minutes later and again impaled with a chosen delay. About one hour after the first impalement, the TNC is impaled to the bottom of the slice and completely withdrawn after half an hour. Finally, profiles of the field potential are probed in 20 µm steps during an impalement, a withdrawal and another impalement.

### Results:

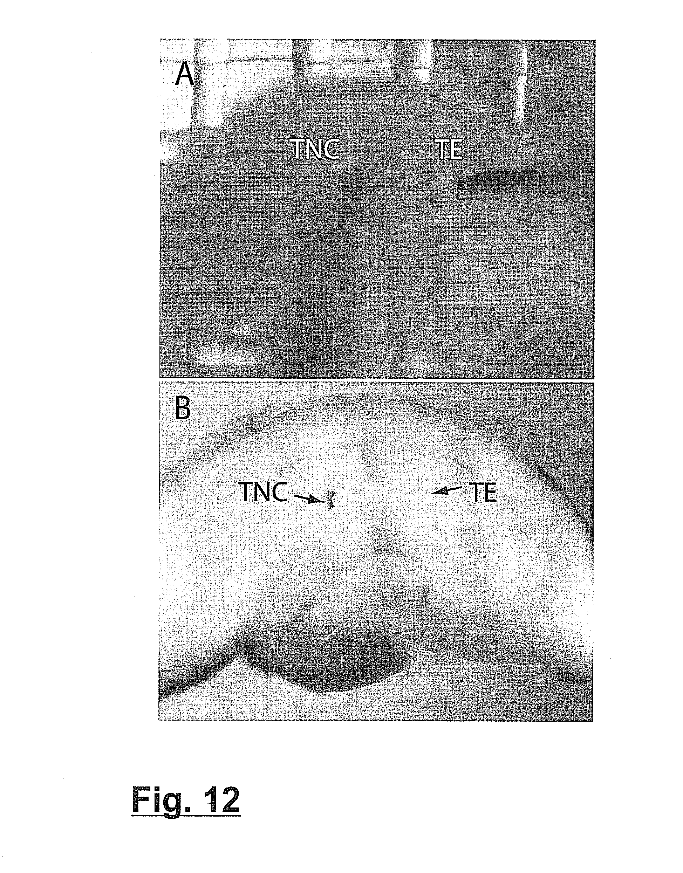

An acute slice with a thickness of 800 µm was held on the agar layer and a TNC was impaled into stratum radiatum of the CA1 region with the transistors facing the incoming Schaffer collateral. A tungsten electrode was placed in the Schaffer collateral as illustrated in Fig. 12A. The same slice is shown in Fig 12B after the experiments when the TNC and the tungsten electrodes were withdrawn.

An experiment with recordings of four transistors is shown in Fig. 13A when the Schaffer collateral was stimulated 26 min after impalement. The first transistor was 600 µm above the bottom of the slice with the other transistors nearer to its surface. After a stimulation artefact and a fiber volley, the voltage transients of the transistors are observed that have the typical shape of extracellular field potentials due to excitatory synaptic currents [22]. The amplitude recorded by the first transistor was around -1.6 mV whereas the signals of the other transistors were smaller. In test experiments, a micropipette electrode was held with its tip close to the transistors of a TNC during impalement. The recordings were almost identical to the transistor signals.

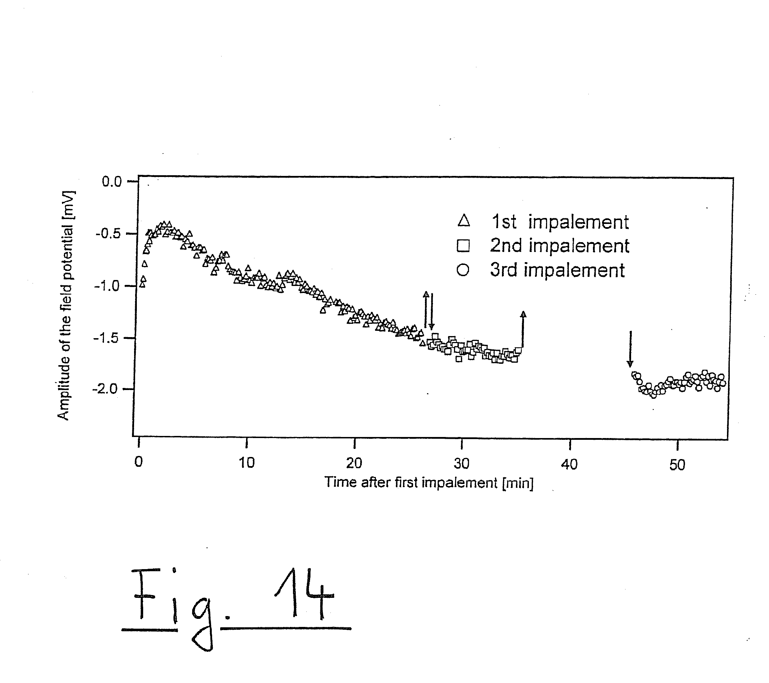

The transistor recordings changed with the duration of impalement. Fig. 13B shows four recordings of the first transistor in the same experiment. The signal decreases within the first two minutes after impalement and subsequently increases within 26 minutes. The amplitude of the field potential is plotted in Fig. 14 versus the duration of impalement. The decrease and increase of the amplitude was observed with similar amplitudes in all experiments (n=4). This change of the amplitudes was also recorded with a micropipette electrode that was impaled together with a TNC. When a micropipette was impaled without TNC, high signal amplitudes were recorded immediately upon the first impalement.

When a TNC was withdrawn and impaled again to the same depth at the same position, there was no slow adaptation of the recorded signals. Field potentials with high amplitudes were observed without delay as shown in Fig. 14. Even after a second withdrawal and another impalement after ten minutes, high amplitudes were recorded.

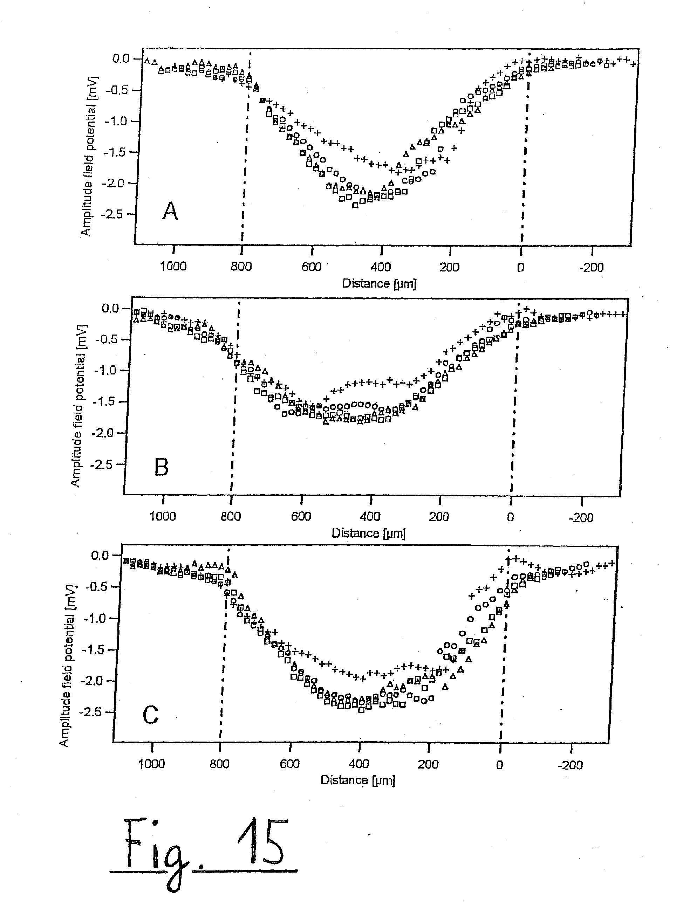

The stability of large field potentials was also observed when profiles of field potentials were recorded across the slice. Before that experiment, the TNC was impaled across the whole slice, kept for half an hour in a penetrated position and completely withdrawn. Then the TNC was impaled, withdrawn and impaled again at the same place in steps of 20 µm. At each depth we stimulated the slice three times and determined the field potential by averaging. The profile of the amplitudes is plotted in Fig. 15 for all four transistors. All profiles are similar. The amplitudes are large when a transistor is near the centre of the slice and small when a transistor approaches the upper or lower surface of the slice. There is some variation of the profiles for impalement, withdrawal and re-impalement and also some difference of the profiles that are recorded with the transistor near the tip of TNC and the other transistors. Similar results were obtained in different experiments (n = 4).

**Discussion.** Negative field potentials in stratum radiatum of the hippocampus are due to excitatory synaptic currents in the dendrites of pyramidal neurons in CA1 as induced by the Schaffer collaterals (Anderson et al., 1977). Transistors on needle-shaped silicon chips are able to records these field potentials in the central layer of acute slices where the tissue is not damaged by the cutting process. The signals correspond to recordings with impaled micropipette electrodes. They are larger than transistor recordings at the surface of slices with damaged tissue (Stangl & Fromherz, 2008).

A striking issue of the experiments is the adaptation of the slice to the impaled transistor needle chip. The amplitude of the recorded field potential is small upon a first impalement and becomes even smaller within the first two minutes. Subsequently, the signal amplitude is enhanced within an hour to a high stationary level. That enhancement persists when the TNC is withdrawn and impaled again after several minutes. There is a functional consolidation of the tissue-chip interaction that must be assigned to a structural reorganization of the tissue around the needle chip. The result suggests detailed histological studies on the tissue-chip interaction.

After adaptation, a profile of the recorded field potential is observed across the slice with a maximum of the negative amplitude in the center. It is not related with the nature of transistor interfacing, but reflects the typical situation of a brain slice where synaptic current flows to the electrolyte (bath and agar layer) on ground potential. As a simple model, a homogeneous slice of thickness that is held on ground potential at both surfaces is considered. The curvature of the field potential is *d*ϕ ² / dz² = - ρ j, with a resistivity ρ and a current source density j according to the volume conductor theory (Mitzdorf, 1985). With the boundary conditions ϕ(z = 0) = 0 and ϕ(z = h) = 0, a parabolic profile is formed with a maximum ϕ^{max} = ρ j h² / 8 in the center of the slice. It resembles the experiments of Fig. 15 with some deviations that are due to the damaged tissue on both sides of the slice (about 100 µm) (Stangl & Fromherz, 2008) and the finite resistance (50 Ωcm) of the electrolyte. Using the experimental values ϕ^{max} = -2 mV and h = 800 µm, we obtain with ρ = 300 Ωcm (Holsheimer, 1987) a current source density maximum j ≈ - 10 µA / mm³ that is in a proper order of magnitude (Taube & Schwatzkroin, 1988; Brankack et al., 1993).

There are minor differences of the profiles recorded for repeated impalement and withdrawal and also minor differences of the profiles recorded with the first transistor and the other transistors (Fig. 15). These effects must be assigned to the interaction of chip and tissue which is not exactly the same for repeated impalements and withdrawals and for the tip and the shaft of the TNC.

Conclusion. It was demonstrated that a direct interfacing of transistors and intact brain tissue can be implemented using a transistor needle chip, i.e. an embodiment of a neurological interfacing probe according to the invention, in an acute brain slice. The TiO2 surface of the chips provides chemical stability and biocompatibility. The tissue adapts to an impaled needle within one hour such that stable recordings of field potentials are attained. The application of TNCs in the brain of living animals appears to be feasible.

### Abbreviations

CA cornu ammonis
FET field effect transistor
TNC transistor needle chip

For a neurological interfacing probe, having
- a contacting section having a plurality of contacts for electrically connecting the neurological interfacing probe and
- an elongated impalement section extending from the contacting section and being adapted for impaling into neuronal tissue to be interfaced;
- wherein the impalement section has a plurality of interaction sites which each are electrically connected with at least one respective contact of said plurality of contacts by means of at least one respective interconnecting trace extending along the impalement section and which are adapted to electrically interact with the neuronal tissue;
it is proposed that the plurality of interaction sites are provided by semiconductor structures which are formed in semiconductor material and which are adapted to provide at least one of transistor functionality and capacitor functionality for electrically interacting with the neuronal tissue. Additionally and alternatively it is proposed that the interaction sites are covered by at least one insulating layer electrically isolating the interaction sites from the neuronal tissue to provide capacitive coupling between the respective interaction sites and the neuronal tissue.

### References

- Anderson, P., Sundberg, S.H., Sven, O. & Wigström, H. (1977) Specific long-lasting potentiation of synaptic transmission in hippocampal slices. Nature 266, 736-737.
- Brankack J., Stewart, M. & Fox, S.E. (1993) Current source density analysis of the hippocampal theta rhythm: associated sustained potentials and candidate synaptic generators. Brain Res. 615, 310— 327.
- Dingledine, R. (1984) Brain Slices. Plenum Press, New York.
- Donoghue, J. P. (2002) Connecting cortex to machines: recent advances in brain interfaces. Nature Neuroscience 5, 1085-1088.
- Hochberg, L.R., Serruya, M.D., Friehs, G.M., Mukand, J.A., Saleh, M., Caplan, A.H., Branner, A., Chen, D., Penn, R.D. & Donoghue, J.P. (2006) Neuronal ensemble control of prosthetic devices by a human with tetraplegia. Nature 442, 164-171.
- Holsheimer, J. (1987) Electrical conductivity of hippocampal C1 layers and application to current-source density analysis. Exp. Brain Res. 67, 402-410.
- Korte, M., Griesbeck, O., Gravel, C., Carroll, P., Staiger, V., Thoenen, H. & Bonhoeffer, T. (1996) Virus-mediated gene transfer into hippocampal CA1 region restores long-term potentiation in brain-derived neurotrophic factor mutant mice. Proc. Natl. Acad. Sci. USA 93, 12547-12552.
- Kralik, J.D., Dimitrov, D.F., Krupa, D.J., Katz, D.B., Cohen, D. & Nicolelis, M.A.L. (2001) Techniques for long-term multisite neuronal ensemble recordings in behaving animals. Methods 25, 121-150.
- Lebedev, M. A. & Nicolelis, M.A.L. (2006) Brain-machine interfaces: past, present and future. Trends Neurosci. 29,536-546.
- Miller, C.A., Robinson, B.K., Hetke, J.F., Abbas, P.J. & Nourski, K.V. (2004) Feasibility of using silicon-substrate recording electrodes within the auditory nerve. Hear. Res. 198, 48-58.
- Mitzdorf, U. (1985) Current-source density method and application in cat cerebral cortex: investigation of evoked field potentials and EEG phenomena. Physiol. Rev. 65, 37-100.
- Nicolelis, M.A.L., Dimitrov, D., Carmena, J.M., Crist, R., Lehew, G., Kralik, J.D. & Wise, S.P. (2003) Chronic, multisite, multielectrode recordings in macaque monkeys. Proc. Natl. Acad. Sci. USA 100, 11041-11046.
- Normann, R.A., Maynard, E.M., Rousche, P.J. & Warren, D.J. (1999) A neural interface for a cortical vision prosthesis. Vision Res. 39, 2577-2587.
- Schoen, I. & Fromherz, P. (2008) Extracellular stimulation of mammalian neuron through repetitive activation of Na+ channels by weak capacitive currents on a silicon chip. J. Neurophysiol. 100, 346-357.
- Schwartz, A.B., Cui, X.T., Weber, D.J. & Moran, D.W. (2006) Brain-controlled interfaces: Movement restoration with neural prosthetics. Neuron 52, 205-220.
- Sowa, M.J., Littau, M.E., Pohray, V. & Cecchi, J.L. (2000) Fluorocarbon polymer deposition kinetics in a low-pressure, high density, inductively coupled plasma reactor. J. Vac. Sci. Technol. A 18, 2122-2129.
- Stangl, C., & Fromherz, P. (2008) Neuronal field potential in acute hippocampus slice recorded with transistor and micropipette electrode. Eur. J. Neurosci. 27, 958-964.
- Syau, T., Baliga, B. J. & Hamaker, R. W. (1991) Reactive ion etching of silicon trenches using SF6/O2 gas mixtures. J. Electrochem. Soc. 138, 3076-3081.
- Taube, J. S. & Schwartzkroin, P. A. (1988) Mechanisms of long-term potentiation: a current-source density analysis. J. Neurosci. 8, 1645-1655.
- Voelker, M. & Fromherz, P. (2005) Signal transmission from individual mammalian nerve cell to field-effect transistor. Small 1, 206-210.
- Weis, R., Müller, B., & Fromherz, P. (1996) Neuron adhesion on a silicon chip probed by an array of field-effect transistors. Phys. Rev. Left. 76, 327-330.
- Weis, R. & Fromherz, P. (1997) Frequency dependent signal transfer in neuron transistors. Phys. Rev. E 55, 877-889.
- A. Stett, B. Müller and P. Fromherz, Physical Review E, 55, 1779 (1997).
- M. Hutzler and P. Fromherz, European Journal of Neuroscience, 19, 2231 (2004).
- C. Stangl and P. Fromherz, European Journal of Neuroscience, 27, 958 (2008).
- L. Galvani, in Ostwald's Klassiker der exakten Wissenschaften, Thun, Frankfurt am Main (1791).
- B. Franklin, Philosophical Transactions (1 683-1775), 50,481(1757). 6. J. S. T. Gehier, Physikalisches Wörterbuch oder Versuch einer Erklärung der vornehmsten Begriffe und Kunstwörter der Naturlehre, Frommann-Holzboog, Stuttgart-Bad Cannstatt (1787-1796).
- C. Goulding, Journal of the Royal Society of Medicine, 95, 257 (2002).
- R. C. Gesteland, B. Howland, J. Y. Lettvin and W. H. Pitts, Proceedings of the Institute of Radio Engineers, 47, 1856 (1959).
- H. Altner and J. Boeckh, Zeitschrift für vergleichende Physiologie, 55, 299 (1967).
- M. Lepselte, Bell System Technical Journal, 45, 233 (1966).
- R. P. Feynman, Plenty of Room at the Bottom, California Institute of Technology (1959).
- Silicon Microelectrode array Research Product Catalog & Manual, NeuroNexus Technologies (2008).
- P. Fromherz, Brain on Line? The feasibility of a neuron-silicon-junction (1985).
- P. Fromherz, A. Offenhausser, T. Vetter and J. Weis, Science, 252, 1290(1991).
- P. Fromherz and A. Stett, Physical Review Letters, 75, 1670 (1995).
- M. Jenkner, B. Müller and P. Fromherz, Biological Cybernetics, 84, 239(2001).
- B. J. Besi and P. Fromherz, Biophysical Journal, 80, 100A (2001).
- G. Zeck and P. Fromherz, Proceedings of the National Academy of Sciences of the United States of America, 98, 10457 (2001).
- R. Zeitler, Elektrisches Interfacing von Schneckenneuronen an CMOS Chips, TU München, München (2004).
- B. Besl, Elektrische Kopplung von Hirnschnitten mit Feldeffekttransistoren, TU München, München (2001).
- F. Wallrapp, Hoch-K-Materialien in der Elektrolyt-Isolator/Silizium-Konfiguration: Charakterisierung und Anwendung in der Bioelektronik, TU München, München (2006).
- I. Schön, Extrazelluläre Stimulation von lonenkanälen und Nervenzellen mittels Elektrolyt/Oxid/Silizium-Kondensatoren, TU München (2006). 23. P. Fromherz, European Biophysics Journal with Biophysics Letters, 28, 254 (1999).
- M. Brittinger, Elektrische und chemische Signalübertragung in Zell-Transistor-Chips mit lonenkanälen, TU München, München (2004).
- C. Stangl, Elektrische Kopplung akuter Hirnschnitte an Feldeffekttransistoren, TU München, München (2004).
- M. Ulbrich, Öffnen von lonenkanälen durch kapazitive Kopplung in Zellmembranen auf Siliziumchips, TU München, München (2003).
- K. D. Wise, J. B. Angell and A. Starr, IEEE Transactions on Biomedical Engineering, BM17, 238 (1970).
- P. K. Campbell, K. E. Jones, R. J. Huber, K. W. Horch and R. A. Nonnann, IEEE Transactions on Biomedical Engineering, 38, 758 (1991).
- S. Kim, P. Tathireddy, R. A. Normann and F. Solzbacher, IEEE Transactions on Neural Systems and Rehabilitation Engineering, 15, 493 (2007).
- K. H. Leong, G. Ensell, P. Wall and R. S. Pickard, Biosensors & Bioelectronics, 5, 303 (1990).
- K. Najafi, K. D. Wise and T. Mochizuki, IEEE Transactions on Electron Devices, 32, 1206 (1985*).*
- D. J. Edell, IEEE Transactions on Biomedical Engineering, 33, 203 (1986).
- K. Najafi and K. D. Wise, IEEE Journal of Solid-State Circuits, 21, 1035 (1986). 34. G. Gerlach and W. Dotzel, Grundlagen der Mikrosystemtechnik, Fachbuchverlag Leipzig, Leipzig (1997).
- G. Ensell, D. J. Banks, D. J. Ewins, W. Balachandran and P. R. Richards, Journal of Microelectromechanical Systems, 5, 117 (1996).
- D. J. Anderson, K. Najafi, S.I. Tanghe, D. A. Evans, K. L. Levy, J. F. Hetke, X. L. Xue, J. J. Zappia and K. D. Wise, IEEE Transactions on Biomedical Engineering, 36, 693 (1989).
- P. Norlin, M. Kindlundh, A. Mouroux, K. Yoshida and U. G. Hofmann, Journal of Micromechanics and Microengineering, 12, 414 (2002).
- A. Blau, C. Ziegler, M. Heyer, F. Endres, G. Schwitzgebel, T. Matthies, T. Stieglitz, J. U. Meyer and W. Gopel, Biosensors & Bioelectronics ,12, 883 (1997).
- K. D. Wise, D. J. Anderson, J. F. Hetke, D. R. Kipke and K. Najafi, Proceedings of the IEEE, 92, 76 (2004).
- H. Seidel, L. Csepregi, A. Heuberger and H. Baumgartel, Journal of the Electrochemical Society, 137, 3626 (1990).
- G. Ensell, D. J. Banks, P. R. Richards, W. Balachandran and D. J. Ewins, Medical & Biological Engineering & Computing ,38, 175 (2000).
- D. T. Kewley, M. D. Hills, D. A. Borkholder, I. E. Opris, N. I. Maluf, C. W. Storment, J. M. Bower and G. T. A. Kovacs, Sensors and Actuators a-Physical, 58, 27 (1997).

- W. Fussel, M. Schmidt, H. Angermann, G. Mende and H. Flietner, Nuclear Instruments & Methods in Physics Research Section a-Accelerators Spectrometers Detectors and Associated Equipment, 377,177 (1996).
- J.K. Chapin, K. A. Moxon, R. S. Markowitz and M. A. L. Nicolelis, Nature Neuroscience, 2, 664 (1999).
- M. S. Schwartz, S. R. Otto, R. V. Shannon, W. E. Hitselberger and D. E. Brackmann, Neurotherapeutics, 5, 128 (2008).
- N. Klauke, G. L. Smith and J. Cooper, Biophysical Journal, 85, 1766 (2003).
- L. J. Breckenridge, R. 3. A. Wilson, P. Connolly, A. S. G. Curtis, J. A. T. Dow, S. E. Blackshaw and C. D. W. Willcinson, Journal of Neuroscience Research, 42, 266 (1995).
- C. Q. Huang, R. K. Shepherd, P. M. Seligman and O. M. Clark, Hearing Research, 116, 55 (1998).
- M. P. Maher, J. Pine, 3. Wright and Y. C. Tai, Journal of Neuroscience Methods, 87, 45 (1999).
- D. H. Szarowski, M. D. Andersen, S. Retterer, A. J. Spence, M. Isaacson, H. 0. Craighead, I. N. Turner and W. Sham, Brain Research ,983, 23 (2003).
- F. M. Martinez-Santiesteban, S. D. Swanson, D. C. Noll and D. 3. Anderson, Physics in Medicine and Biology ,52, 2073 (2007).
- M. Hutzler, Elektrisches Interfacing von Halbleiterchips an kultivierten Hirnschnitten, TU München, München (2003).
- K. R. Williams, K. Gupta and M. Wasilik, Journal of Microelectromechanical Systems, 12, 761 (2003).
- J. Brugger, G. Beijakovic, M. Despont, H. Biebuyck, N. F. de Rooij and P. Vettiger, Sensors and Actuators a-Physical, 70, 191 (1998).
- A. Reisman, M. Berkenblit, S. A. Chan, F. B. Kaufman and D. C. Green, Journal of the Electrochemical Society,126, 1406 (1979).
- B. Straub, Gestützte Lipidmembran auf Feldeffekttransistor, TU München, München (1996).
- E. Bassous, H. H. Taub and L. Kubn, Applied Physics Letters, 31, 135 (1977).
- F. Laermer and A. Schilp, Method of anisotropically etching silicon, B. G. Robert (Editor), United States (1996).
- R. Legtenberg, H. Jansen, M. Deboer and M. Elwenspoek, Journal of the Electrochemical Society, 142, 2020 (1995).
- R. Knizikevicius, Sensors and Actuators a-Physical, 132, 726 (2006).
- R. J. Belen; S. Gomez, D. Cooperberg, M. Kiehlbauch and E. S. Aydil, Journal of Vacuum Science & Technology A, 23, 1430 (2005).
- S. Grigoropoulos, E. Gogolides, A. D. Tserepi and A. G. Nassiopoulos, Journal of Vacuum Science & Technology B, 15, 640 (1997).
- R. Dagostino, F. Cramarossa, V. Colaprico and R. Dettole, Journal of Applied Physics, 54, 1284 (1983).
- H. Zou, Microsystem Technologies-Micro-and Nanosystems-Information Storage and Processing Systems, 10, 603 (2004).
- H. Jansen, M. Deboer, R. Legtenberg and M. Elwenspoek, Journal of Micromechanics and Microengineering, 5, 115 (1995).
- A. A. Ayon, R. L. Bayt and K. S. Breuer, Smart Materials & Structures, 10, 1135 (2001).
- Y. Iriyama and M. Noda, Journal of Polymer Science Part a-Polymer Chemistry, 36, 2043 (1998).
- H. Kobayashi, Asuha, I. Sung-Soon, M. Tanaka, S. Imai and M. Takahashi, Surface Science,600, 2523 (2006).
- D. W. Hess, IBM Journal of Research and Development, 43, 127 (1999).
- P. Smeys, Local Oxidation Of Silicon for Isolation, Stanford University, Stanford (1996).
- J. J. Finley and H. F. Gilleiy, Alkali metal diffusion barrier layer, P. I. I. (US) Editor (1998).
- R. Kaul, Chemical Synapses on Semiconductor Chips, TU München, München (2007).
- P. Bonifazi and P. Fromherz, Advanced Materials, 14, 1190 (2002).
- M. Völker, Detektion von Aktionspotentialen einzelner Säugetierneurone mit rauscharmen Feldeffekttransistoren nahe der thermodynamischen Meßgrenze, TU München (2006).
- F. Walrapp, Halbleiter mit Hoch-DK-Beschichtung zur Stimulation von Nervenzellen, TU München, München (2003).
- S. M. Sze, Physics of semiconductor devices, John Wiley & Sons, New York (1981).
- N. I. Syed, A. G. M. Bullock and K. Lukowiak, Science, 250, 282 (1990).
- I. Schoen and P. Fromherz, Biophysical Journal, 92, 1096 (2007).
- J. C. Weaver and Y. A. Chizmadzhev, Bioelectrochemistry and Bioenergetics, 41, 135 (1996).
- V. S. Polikov, P. A. Tresco and W. M. Reichert, Journal of Neuroscience Methods, 148, 1 (2005*).*
- D. Purves, Neuroscience, Sinauer, Sunderland, Mass. (1997).
- T. J. Blanche, M. A. Spacek, J. F. Hetke and N. V. Swindale, Journal of Neurophysiology, 93, 2987 (2005*).*
- H. Seidel, L. Csepregi, A. Heuberger and H. Baumgartel, Journal of the Electrochemical Society, 137, 3612 (1990).
- N. Layadi, J. I. Colonell and J. T. C. Lee, Bell Labs Technical Journal, 4,155 (1999).
- C. Cardinaud, M. C. Peignon and P. Y. Tessier, Applied Surface Science, 164, 72 (2000).
- R. W. Calm, P. Haasen and E. J. Kramer, Materials Science and Technology, VCH Weinheim (1996).
- L. M. Roylance and J. B. Angell, IEEE Transactions on Electron Devices, 26, 1911 (1979).
- O. Tabata, R. Asahi, H. Funabashi, K. Shimaoka and S. Sugiyama, Sensors and Actuators a-Physical, 34, 51 (1992).
- R. M. Finne and D. L. Klein, Journal of the Electrochemical Society, 114,965 (1967).
- U. Hilleringmann, Mikrosystemtechnik, B.G. Teubner Verlag, Wiesbaden (2006).
- W. Kern and D. A. Puotinen, RCA Review, 31, 187 (1970).
- M. Schulz, Microelectronic Engineering, 40, 113 (1998).
- S. Wolf and R. N. Tauber, Silicon Processing for the VLSI Era, Lattice Press, Sunset Beach (1986).
- H. J. Schliwinski, U. Scbnakenberg, W. Windbracke, H. Neff and P. Lange, Journal of the Electrochemical Society, 139, 1730 (1992).
- O. Stetter, Stimulation von Säugetier-Neuronen mit TiO2/Al2O3/Si-Kondensatoren, LMU München, München (2008).
- T. E. Seidel, D. J. Lischner, C. S. Pai, R. V. Knoell, D. M. Maher and D. C. Jacobson, Nuclear Instruments & Methods in Physics Research Section B-Beam Interactions with Materials and Atoms, 7-8, 251 (1985).
- J. Aarik, A. Aidla, T. Uustare and V. Sammelselg, Journal of Crystal Growth, 148, 268 (1995).
- M. V. HhajjiHassan, V. Chodavarapu, S. Musallam, NeuriMEMS: Neural Probe Microtechnologies. Sensors, 2008, 8(10), 6704-6726.
- D.H.Szarowski et al., Brain responses to micro-machined silicon devices. Brain Research, 2003. 983(1-2), 23-25.
- P.J. Rousche, R.A. Normann, Chronic recording capability of the Utah Intracortical Electrode Array in cat sensory cortex. Journal of NeuroScience Methods, 1998, 82(1), 1-15.
- D.D. Elsberry, M.T.R. Techniques for treating neurodegenerative disorders by infusion of nerve growth factors into the brain, 2000.
- B. BesI and P. Fromherz, Transistor array with organotypic brain slice: field potential records and synaptic currents. European Journal of Neuroscience, 2002, 15, 999-1005.

## Claims

1. Neurological interfacing probe (10; 10'), having
- a contacting section having a plurality of contacts for electrically connecting the neurological interfacing probe (10; 10') and
- an elongated impalement section extending from the contacting section and being adapted for impaling into neuronal tissue (18) to be interfaced;
- wherein the impalement section (12; 12') has a plurality of interaction sites (14; 14') which each are electrically connected with at least one respective contact of said plurality of contacts by means of at least one respective interconnecting trace (16; 16') extending along the impalement section (12; 12') and which are adapted to electrically interact with the neuronal tissue (18), and
- wherein at least the impalement section comprises
semiconductor material;
**characterized in that** the plurality of interaction sites (14; 14') are provided by semiconductor structures which are formed in the semiconductor material and which are adapted to provide at least one of transistor functionality and capacitor functionality for electrically interacting with the neuronal tissue (18).

2. Neurological interfacing probe according to claim 1,
wherein the interaction sites (14; 14') are covered by at least one insulating layer electrically isolating the interaction sites (14; 14') from the neuronal tissue (18).

3. Neurological interfacing probe, having
- a contacting section having a plurality of contacts for electrically connecting the neurological interfacing probe (10; 10') and
- an elongated impalement section (12; 12') extending from the contacting section and being adapted for impaling into neuronal tissue (18) to be interfaced;
- wherein the impalement section (12; 12') has a plurality of interaction sites (14; 14') which each are electrically connected with at least one respective contact of said plurality of contacts by means of at least one respective interconnecting trace (16; 16') extending along the impalement section (12; 12') and which are adapted to electrically interact with the neuronal tissue (18);
**characterized in that** the interaction sites (14; 14') are covered by at least one insulating layer electrically isolating the interaction sites (14; 14') from the neuronal tissue (18) to provide capacitive coupling between the respective interaction sites (14; 14') and the neuronal tissue (18).

4. Neurological interfacing probe according to claim 3,
wherein a least one interaction site (14; 14') is adapted to provide transistor functionality for electrically interacting with the neuronal tissue (18) by means of said capacitive coupling,
and/or
wherein at least one interaction site (14; 14') is adapted to provide capacitor functionality for electrically interacting with the neuronal tissue (18) by means of said capacitive coupling,
or/and
wherein at least the impalement section (12; 12') comprises semiconductor material and at least one interaction site (14; 14') is provided by a semiconductor structure which is formed in the semiconductor material and which is adapted to provide at least one of transistor functionality and capacitor functionality for electrically interacting with the neuronal tissue (18).

5. Neurological interfacing probe according to one of claims 1 to 4,
wherein at least a subset of the interaction sites (14; 14') is provided in the form of field effect transistors having at least a drain section (94), a channel section (90) and a source section (92), wherein the drain section (94) and the source section (92) are connected with a respective contact of the contacting section by means of a respective interconnecting trace (16; 16'), and
wherein the channel section (9) or a gate section of the field effect transistor serves to electrically couple the field effect transistor with the neuronal tissue (18).

6. Neurological interfacing probe according to one of claims 1 to 5,
wherein at least a subset of the interaction sites (14; 14') is provided in the form of electrode sections which are connected with a respective contact of the contacting section by means of a respective interconnecting trace (16; 16') and are adapted to serve as capacitor for electrically coupling with the neuronal tissue (18).

7. Neurological interfacing probe according to claim 5 or 6,
wherein the electrode sections or/and the drain sections (94) or/and the source sections (92) are provided by highly doped semiconductor material sections arranged between less highly doped or differently doped semiconductor material.

8. Neurological interfacing probe according to one of claims 1 to 7,
wherein the interconnecting traces (16; 16') are provided by highly doped semiconductor material sections arranged between less highly doped or differently doped semiconductor material.

9. Manufacturing method for manufacturing a neurological interfacing probe according to one of the preceding claims,
wherein the contacting section and the impalement section (12; 12') are integrally etched from a semiconductor substrate (26) after the formation of the semiconductor structures.

10. Manufacturing method according to claim 9,
wherein the semiconductor substrate (26) has an etch stop layer (30) for at least one etching step, preferably at least one of a first and a second etching step.

11. Manufacturing method according to claim 10,
wherein the semiconductor substrate (26) is a Silicon-on-Insulator (SOI) wafer having a buried oxide layer which serves as etch stop layer (30).

12. Manufacturing method according to one of claims 9 to 11,
wherein the contacting section and the impalement section (12; 12') are etched using a multi-step-etching process, with at least a first etching step being performed on a first side (A) of the semiconductor substrate (26) and at least a second etching step being performed on a second side (B) of the semiconductor substrate (26), on which the semiconductor structures are formed.

13. Manufacturing method according to claim 12,
wherein the first etching step is based on a wet etching process, preferably an EDP etching process
or/and
wherein the second etching step is based on a dry etching process, preferably a plasma etching process.

14. Manufacturing method according to any of claims 9 to 13,
wherein a multi-step structuring process is carried out on a/the second side (B) of the semiconductor substrate (26), preferably preceding a/the first etching step,
wherein the multi-step structuring process comprises at least one of the following substeps:
S1) implantation of a dopant on the second side (B) of the
semiconductor substrate (26) such that a plurality of highly doped semiconductor sections (40) are formed within the semiconductor substrate (26);
S2) formation of an silicon dioxide layer (44) on the second side (B)
of the semiconductor substrate (26) followed by partial etching of the silicon dioxide layer (44) such that a silicon oxide section (50) serving as gate oxide of a field effect transistor is formed from the silicon dioxide layer (44);
S3) deposition of an insulating layer (56) on the second side (B) of
the semiconductor substrate (26);
S4) deposition of a metal layer (62) on the second side (B) of the
semiconductor substrate (26) followed by partial removal of the metal layer (62) such that metal sections are formed from the metal layer serving as contacts.

15. Manufacturing method according to any of claims 9 to 14,
wherein a/the first etching step is part of a multi-step processing of the semiconductor substrate (26) which comprises at least one of the following steps:
F1) deposition of a protection layer (64), preferably a polyimide
layer, on a/the second side (B) of the semiconductor substrate (26);
F2) deposition of a silicon dioxide layer (66) on a/the first side (A)
of the semiconductor substrate (26) followed by partial etching of the silicon dioxide layer (66) such that a silicon dioxide section partially covering the semiconductor substrate (26) is formed;
F3) partial removal of the semiconductor substrate (26) by etching,
preferably wet etching such that the buried etch stop layer (30) is exposed on the first side (A) after removal of the semiconductor substrate (26);
F4) removal of the protection layer if deposited in step F1).

16. Manufacturing method according to any of claims 9 to 15,
wherein a/the second etching step is part of a multi-step processing of the semiconductor substrate which comprises at least one of the following steps:
G1) deposition of a a protection layer (72), preferably a polyimide
layer, on top of a/the second side (B) of the semiconductor substrate (26);
G2) removal of the semiconductor substrate (26) by plasma etching
such that the buried etch stop layer (30) is exposed on the second side (B) after removal of the semiconductor substrate (26);
G3) removal of the protection layer if deposited in step G1);
G4) separation of the neurological interfacing probe (10; 10') by
removing or braking the etch stop layer (30).

17. Method for measuring brain activity or/and for inducing brain activity in a brain, preferably the brain of a living creature, comprising the step of impaling the impalement section (12; 12') of the neurological interfacing probe (10; 10') according to one of claims 1 to 8 in the brain and the steps of measuring or/and inducing action potentials of neuronal tissue (18) of the brain by electrical means using the contacts of the contacting section.
